(19) Europäisches Patentamt
European Patent Office
Office européen des brevets

(11) **EP 4 285 908 A2**

(12) **EUROPEAN PATENT APPLICATION**

(43) Date of publication:
**06.12.2023 Bulletin 2023/49**

(21) Application number: **23204417.2**

(22) Date of filing: **27.03.2018**

(51) International Patent Classification (IPC):
***A61K 31/5575*** *(2006.01)*

(52) Cooperative Patent Classification (CPC):
(C-Sets available)
**A61K 9/0048; A61J 1/00; A61K 9/08; A61K 31/472; A61K 31/4725; A61K 31/5575; A61K 47/02; A61K 47/10; A61K 47/12; A61K 47/186; A61K 47/34; A61P 27/02; A61P 27/06** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**KH MA MD TN**

(30) Priority: **27.03.2017 JP 2017060582**
**27.03.2017 JP 2017060585**
**27.03.2017 JP 2017060586**
**28.04.2017 JP 2017089205**

(62) Document number(s) of the earlier application(s) in accordance with Art. 76 EPC:
**18775859.4 / 3 603 642**

(71) Applicant: **Alcon Inc.**
**1701 Fribourg (CH)**

(72) Inventor: **YAMAKITA, Yoshihiro**
**Fuji-shi Shizuoka, 417-8650 (JP)**

(74) Representative: **Elkington and Fife LLP**
**Prospect House**
**8 Pembroke Road**
**Sevenoaks, Kent TN13 1XR (GB)**

Remarks:
This application was filed on 18-10-2023 as a divisional application to the application mentioned under INID code 62.

# (54) PHARMACEUTICAL PREPARATION

(57) To establish a technique for stably formulating netarsudil or a salt thereof or a solvate thereof as an ophthalmic agent or the like. Use of latanoprost and a container formed of polypropylene or polyethylene is provided, for suppressing the content reduction of netarsudil or a salt thereof or a solvate thereof in a pharmaceutical preparation comprising an aqueous composition comprising said latanoprost and said netarsudil or a salt thereof or a solvate thereof, wherein the aqueous composition is housed in said container formed of polypropylene or polyethylene.

EP 4 285 908 A2

(52) Cooperative Patent Classification (CPC): (Cont.)

C-Sets
**A61K 31/472, A61K 2300/00;**
**A61K 31/4725, A61K 2300/00;**
**A61K 31/5575, A61K 2300/00**

## Description

Technical Field

The present invention relates to pharmaceutical preparations and the like.

Background Art

Several isoquinoline-6-amino derivatives have hitherto been known to be useful in prevention and treatment of eye diseases such as ocular hypertension and glaucoma.

Specifically, for example, a compound represented by the following structural formula:

(Chemical Name: [4-[(2S)-3-amino-1-(isoquinolin-6-ylamino)-1-oxopropan-2-yl]phenyl]methyl 2,4-dimethylbenzoate), International Nonproprietary Name: netarsudil, hereinafter sometimes referred to as "netarsudil"), also known as AR-13324, has been reported to have pharmacological effects such as Rho kinase inhibitory effect and norepinephrine transporter inhibitory effect, and be useful in prevention and treatment of eye diseases such as ocular hypertension and glaucoma (for example, Patent Literature 1, and Non Patent Literatures 1 and 2).

Similarly, a compound represented by the following structural formula:

(Chemical Name: rac-(2R)-2-(dimethylamino)-N-(1-oxo-1,2-dihydroisoquinolin-6-yl)-2-(thiophen-3-yl)acetamide), International Nonproprietary Name: verosudil, hereinafter sometimes referred to as "verosudil"), also known as AR-12286, has been reported to have Rho kinase inhibitory effect and be useful in prevention and treatment of eye diseases such as ocular hypertension (for example, Patent Literature 2, and Non Patent Literatures 1 and 3).

Accordingly, it is very useful to establish a technique for stably formulating these isoquinolin-6-amino derivatives, for example, as an ophthalmic agent or the like.

Meanwhile, Patent Literature 3 states that a composition containing netarsudil or verosudil was prepared in a 150 mL plastic container. However, in the literature, each composition was prepared for use in pharmacological studies. The literature does not describe the stability of netarsudil and verosudil in the composition at all, neither describes the material and characteristics of the container at all

Citation List

Patent Literature

Patent Literature 1: JP-A-2012-525386
Patent Literature 2: WO2009/091898
Patent Literature 3: JP-A-2016-515520

Non Patent Literature

Non Patent Literature 1: Bacharach J. et al., Ophthalmology 122(2) 302-7 (2015)
Non Patent Literature 2: Sturdivant JM. et al., Bioorg Med Chem Lett. 26(10) 2475-80 (2016)
Non Patent Literature 3: Williams RD. et al., Am- J-Ophthalmol. 152(5) 834-41 (2011)

SUMMARY OF THE INVENTION

An object of the present invention is to establish a technique for stably formulating an isoquinolin-6-amino derivative such as netarsudil or verosudil as an ophthalmic agent or the like.

Solution to Problem

An ophthalmic agent or the like is usually a composition containing water (an aqueous composition). Thus, the present inventor prepared an aqueous composition comprising an isoquinolin-6-amino derivative such as netarsudil and housed the prepared composition in a container formed of a polyolefin resin to check its storage property. As a result, it was unexpectedly revealed that the content of the isoquinolin-6-amino derivative is reduced after storage at high temperature, which content reduction is caused presumably by adsorption to the resin.

Therefore, the present inventor conducted further extensive studies to suppress the content reduction of the isoquinolin-6-amino derivative in an aqueous composition. Consequently, the inventor has found that when the aqueous composition comprising the isoquinolin-6-amino derivative is adapted to further contain one or more components selected from the group consisting of an acid, a quaternary ammonium type surfactant, a polyhydric alcohol and a prostaglandin compound and housed in a container formed of a polyolefin resin, the content reduction is suppressed and a pharmaceutical preparation having improved stability can be obtained, and thus completed the present invention.

Namely, the present invention provides a pharmaceutical preparation comprising an aqueous composition comprising the following components (A) and (B):

(A) a compound represented by the following formula (1)

R1 N A H N O R3 N R2 (1)

(in the formula, $R_1$ and $R_2$ each independently represent a hydrogen atom or a $C_1$-$C_4$ alkyl group,
$R_3$ represents a hydrogen atom or a hydroxy group,
"A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and
the formula (1) also includes a tautomer thereof)
or a salt thereof or a solvate thereof; and

(B) one or more selected from the group consisting of an acid, a quaternary ammonium type surfactant, a polyhydric alcohol and a prostaglandin compound,
wherein the aqueous composition is housed in a container formed of a polyolefin resin.

Effects of the Invention

According to the present invention, it is possible to improve the stability of isoquinolin-6-amino derivatives exemplified by netarsudil in an aqueous composition.

Description of Embodiments

<Component (A)>

In the present specification, "a compound represented by the formula (1):

(1)

(in the formula,

$R_1$ and $R_2$ each independently represent a hydrogen atom or a $C_1$-$C_4$ alkyl group,
$R_3$ represents a hydrogen atom or a hydroxy group,
"A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and
the formula (1) also includes a tautomer thereof) or a salt thereof or a solvate thereof" includes not only a compound represented by the formula (1) itself but also a salt or solvate thereof.

By the way, examples of the salt of the compound represented by the formula (1) are not particularly limited as long as those are pharmaceutically acceptable salts. Specific examples of the salt include an inorganic acid salt such as a hydrochloride, a sulfate, a nitrate, a hydrofluoride, or a hydrobromide; an organic acid salt such as an acetate, a tartrate, a lactate, a citrate, a fumarate, a maleate, a succinate, a methanesulfonate, an ethanesulfonate, a benzenesulfonate, a toluenesulfonate, a naphthalenesulfonate, or a camphorsulfonate, and the hydrochloride and the methanesulfonate are preferred.
Examples of the solvate of the compound represented by the formula (1) or a salt thereof include a hydrate and an alcohol solvate.
Furthermore, when an asymmetric carbon exists in a chemical structure of the compound represented by the formula (1), various stereoisomers can exist, but the configuration of the compound represented by the formula (1) is not particularly limited, and the compound may be a single stereoisomer or a mixture of various stereoisomers in any proportions.
In the specification, when an asymmetric carbon exists in a chemical structure of the compound represented by various formulae, the compound represented by the formula encompasses all of a variety of single stereoisomers and mixtures of the stereoisomers in any proportions, unless otherwise specifies the configuration. Therefore, compounds represented by the formula for which the configuration is not especially specified may be a single stereoisomer or a mixture of various stereoisomers in any proportions.
The compound represented by the formula (1) includes not only a compound directly represented by the formula (1), but also a tautomer of the compound. Specifically, for example, when the formula (1) is the following formula (1a) in which $R_3$ is a hydroxy group, a tautomer thereof (formula (1b)) can exist, then the "compound represented by the formula (1)" includes not only a compound represented by the following formula (1a), but also a compound represented by the formula (1b).

(1 a)

(1 b)

In the present specification, the "$C_1$-$C_4$ alkyl group" means a linear, branched or cyclic alkyl group having 1 to 4 carbon atoms. Specific examples of the "$C_1$-$C_4$ alkyl group" include a methyl group, an ethyl group, a n-propyl group, an isopropyl group, a cyclopropyl group, an-butyl group, a t-butyl group, and an isobutyl group, and the methyl group is preferable.

In the present specification, the "$C_6$-$C_{10}$ aryl group" means an aryl group having 6 to 10 carbon atoms Specific examples of the "$C_6$-$C_{10}$ aryl group" include a phenyl group and a naphthyl group, and the phenyl group is preferable.

In the present specification, the "5 to 10-membered heteroaryl group" means a 5 to 10-membered monocyclic, polycyclic or condensed cyclic aromatic heterocyclic group having 1 to 4 heteroatoms selected from the group consisting of an oxygen atom, a sulfur atom, and a nitrogen atom as a ring-constituting atom(s) - Specific examples of the "5 to 10-membered heteroaryl group" include a furyl group, a thienyl group, a pyrrolyl group, an oxazolyl group, an isoxazolyl group, a thiazolyl group, an isothiazolyl group, an imidazolyl group, a pyrazolyl group, an oxadiazolyl group, a thiadiazolyl group, a triazolyl group, a tetrazolyl group, a pyridyl group, a pyrimidyl group, a pyrazinyl group, a pyridazinyl group, a benzofuranyl group, an isobenzofuranyl group, a benzothienyl group, an indolyl group, an isoindolyl group, an indazolyl group, a benzimidazolyl group, a benzoxazolyl group, a benzisoxazolyl group, a benzothiazolyl group, a benzisothiazolyl group, a benzoxadiazolyl group, a benzothiadiazolyl group, a benzotriazolyl group, a quinolyl group, an isoquinolyl group, a cinnolinyl group, a quinazolinyl group, a quinoxalinyl group, a phthalazinyl group, a naphthyridinyl group, a purinyl group, a pteridinyl group, a furopyridyl group, a thienopyridyl group, a pyrrolopyridyl group, an oxazolopyridyl group, a thiazolopyridyl group, and an imidazopyridyl group, and the thienyl group is preferable.

In the specification, examples of the "substituent" in the "optionally substituted $C_6$-$C_{10}$ aryl group" and "optionally substituted 5 to 10-membered heteroaryl group" specifically include a halogen atom and $-CH_2-OC(=O)-R_5$ (wherein, $R_5$ means a $C_6$-$C_{10}$ aryl group optionally having one to three $C_1$-$C_4$ alkyl group (s) as substituent (s) (e.g., 2,4-dimethylphenyl group), and a chlorine atom and a 2,4-dimethylphenyl carboxymethyl group are preferable.

In the specification, it is preferred that the "optionally substituted $C_6$-$C_{10}$ aryl group" is a 4-chlorophenyl group and a 4-(2,4-dimethylphenyl carboxymethyl)phenyl group.

In the specification, it is preferred that the "optionally substituted 5 to 10-membered heteroaryl group" is a 3-thienyl group.

When $R_3$ is a hydroxy group, the substitution position of $R_3$ is not limited as long as it is on the isoquinoline ring, but it is preferably position 1 of the isoquinoline ring.

In the specification, the "compound represented by the formula (1) or a salt thereof or a solvate thereof" is preferably a compound represented by the following formula (2) :

(2)

or a salt thereof or a solvate thereof; more preferably a compound represented by the following formula (3):

(3)

(netarsudil) (Chemical name: [4-[(2S)-3-amino-1-(isoquinolin-6-ylamino)-1-oxopropan-2-yl]phenyl]methyl 2,4-dimethylbenzoate), International Nonproprietary Name: netarsudil) or a salt thereof or a solvate thereof; and particularly preferably a compound represented by the following formula (4):

(4)

, which is a dimethanesulfonate of the compound represented by the formula (3), (chemical name: [4-[(2S)-3-amino-1-(isoquinolin-6-ylamino)-1-oxopropan-2-yl]phenyl]methyl 2,4-dimethylbenzoate dimethanesulfonate), hereinafter in the specification, it is sometimes referred to as "netarsudil dimesylate").

As another embodiment, the "compound represented by the formula (1) or a salt thereof or a solvate thereof" is preferably a compound represented by the following formula (5) or (5'):

(5)

(5')

or a salt thereof or a solvate thereof; more preferably a compound represented by the following formula (6):

(6)

(verosudil) (Chemical name: rac- (2R) -2- (dimethylamino) -N-(1-oxo-1,2-dihydroisoquinolin-6-yl)-2-(thiophen-3-yl)acetamide), International Nonproprietary Name: verosudil), or a tautomer thereof or a salt of these or a solvate thereof; and particularly preferably a compound represented by the following formula (7):

(7)

, which is a monohydrochloride of the compound represented by the formula (6), (chemical name: rac-(2R)-2-(dimethylamino)-N-(1-oxo-1,2-dihydroisoquinolin-6-yl)-2-(thiophen-3-yl)acetamide monohydrochloride, hereinafter in the specification, it is sometimes referred to as "verosudil monohydrochloride") .

Further, as another embodiment, the "compound represented by the formula (1) or a salt thereof or a solvate thereof" is preferably a compound represented by the following formula (8):

(8)

(3-amino-2-(4-chlorophenyl)-N-(isoquinolin-6-yl)propanamide) or a salt thereof or a solvate thereof

The compound represented by the formula (1) or a salt thereof or a solvate thereof are known compounds, and they can be prepared by a known method. Specifically, for example, the compounds can be prepared with reference to the methods described in Patent Literatures 1 to 3 or Non Patent Literature 2. The contents of these literatures are incorporated herein by reference.

The compound represented by the formula (1) or a salt thereof or a solvate thereof are commercially available, and these commercial products may be used. Specific examples of the commercial products include netarsudil dimesylate (the compound represented by the formula (4)) manufactured by MedChemExpress and by Chemscene LLS; netarsudil monohydrochloride (monohydrochloride of the compound represented by the formula (3)) manufactured by Shanghai Biopharmaleader Co-, Ltd-; and verosudil (a free form of the compound represented by the formula (6)) manufactured by MedKoo Biosciences, Inc.

The content of the compound represented by the formula (1) or a salt thereof or a solvate thereof in the aqueous composition is not particularly limited, and can be appropriately determined depending on the applicable disease and patient's sex, age, symptoms or the like, but, from the viewpoint of obtaining an excellent pharmacological action, the content is preferably from 0.0001 to 5 w/v%, more preferably from 0.001 to 3 w/v%, still more preferably from 0.005 to 2 w/v %, and particularly preferably from 0.01 to 1 w/v% of the compound represented by the formula (1) in terms of a free form relative to the total volume of the aqueous composition.

Particularly, when netarsudil is used as the compound represented by the formula (1), the content is preferably from 0.0001 to 1 w/v%, more preferably from 0.001 to 0.5 w/v%, still more preferably from 0.005 to 0.1 w/v%, and particularly preferably from 0.01 to 0.04 w/v% of netarsudil in terms of a free form relative to the total volume of the aqueous composition, from the viewpoint of obtaining excellent pharmacological action.

Further, when verosudil is used as the compound represented by the formula (1), from the viewpoint of obtaining excellent pharmacological action, the content is preferably from 0.01 to 3.5 w/v%, more preferably from 0.1 to 2.5 w/v%, still more preferably from 0.2 to 1.5 w/v%, and particularly preferably from 0.3 to 0.8 w/v% of verosudil in terms of a free form relative to the total volume of the aqueous composition.

Furthermore, when the compound represented by the formula (8) is used as the compound represented by the formula (1), the content is preferably from 0.0001 to 3 w/v%, more preferably from 0.001 to 2 w/v%, still more preferably from 0.005 to 1 w/v%, and particularly preferably from 0.01 to 0.5 w/v% of the compound represented by the formula (8) in terms of a free form relative to the total volume of the aqueous composition, from the viewpoint of obtaining excellent pharmacological action.

<Component (B)>

The aqueous composition used in the present invention contains (B) one or more selected from the group

consisting of an acid, a quaternary ammonium type surfactant, a polyhydric alcohol and a prostaglandin compound, in addition to the above component (A).

As specifically shown in Test Examples 1 to 6 described later, when the aqueous composition containing the component (A) is housed in a container formed of a polyolefin resin, the content of the compound represented by the formula (1) is reduced after storage at high temperature, which is presumably caused by adsorption to the resin. As a result, it was revealed that the content reduction is suppressed by adding the component (B) to the aqueous composition.

In the specification, the "acid" is not particularly limited, and it may be an organic or inorganic acid. The "acid" is preferably one or more selected from the group consisting of "borate", "phosphate" and "aliphatic carboxylate" from the viewpoint of suppressing the content reduction of the compound represented by the formula (1), and particularly preferably the "borate" and "aliphatic carboxylate".

In the present specification, the "borate" means one or more selected from the group consisting of boric acid and a salt thereof (e.g., an alkali metal salt such as a sodium salt or a potassium salt; an alkaline earth metal salt such as a calcium salt; and an ammonium salt) and a solvate thereof (e.g., a hydrate).

Specific examples of the borate include boric acid, ammonium borate, and borax. In the specification, it is preferred that the "borate" is one or more selected from the group consisting of boric acid and a salt thereof, and more preferably boric acid

All these borates are known compounds, and they may be prepared by a known method or their commercial products may be used

In the present specification, the "phosphate" means one or more selected from the group consisting of phosphoric acid and a salt thereof (e.g., an alkali metal salt such as a sodium salt or a potassium salt; an alkaline earth metal salt such as a calcium salt; and an ammonium salt) and a solvate thereof (e.g., a hydrate).

Specific examples of the phosphate preferably include those listed in Japanese Pharmaceutical Excipients Directory 2016 (published by Yakuji Nippo, Limited) such as phosphoric acid, calcium monohydrogen phosphate, sodium monohydrogen phosphate heptahydrate, trisodium phosphate, dibasic calcium phosphate, dibasic calcium phosphate fine granulated, dibasic sodium phosphate, dibasic sodium phosphate heptahydrate, dibasic sodium phosphate dihydrate, disodium hydrogenphosphate dihydrate, dibasic potassium phosphate, monobasic potassium phosphate, monobasic calcium phosphate hydrate, sodium dihydrogen phosphate dihydrate, sodium dihydrogen phosphate monohydrate, crystalline sodium dihydrogen phosphate dihydrate, calcium pyrophosphate, sodium pyrophosphate, anhydrous sodium pyrophosphate, disodium hydrogen phosphate anhydrous, anhydrous tribasic sodium phosphate, anhydrous dibasic calcium phosphate, anhydrous dibasic calcium phosphate fine granulated, sodium dihydrogen phosphate anhydrous, calcium polyphosphate, or sodium polyphosphate. Among these, from the viewpoint of suppressing the content reduction of the compound represented by the formula (1), one or more selected from the group consisting of phosphoric acid, dibasic sodium phosphate, monobasic potassium phosphate, sodium dihydrogen phosphate dihydrate, sodium dihydrogen phosphate monohydrate, crystalline sodium dihydrogen phosphate dihydrate, disodium hydrogen phosphate anhydrous and sodium dihydrogen phosphate anhydrous are preferable.

All these phosphates are known compounds, so that they may be prepared by a known method or their commercial products may be used. Further, the phosphate in the form of a salt or a complex with another component may be used

In the specification, the "aliphatic carboxylate" means one or more selected from the group consisting of aliphatic carboxylic acid and a salt thereof (e.g., an alkali metal salt such as a sodium salt or a potassium salt; an alkaline earth metal salt such as a calcium salt or a magnesium salt; and an ammonium salt) and a solvate thereof (e.g., a hydrate). The number of carbon atoms in the aliphatic carboxylate is not particularly limited, but preferably from 2 to 20, more preferably from 4 to 18, and particularly preferably from 6 to 16 from the viewpoint of suppressing the content reduction of the compound represented by the formula (1). Further, the carbon chain may be linear or branched, and may be saturated or unsaturated, and furthermore, may include a tertiary amino group and the like between adjacent carbon atoms. Additionally, the number of carboxyl groups in the aliphatic carboxylate is not particularly limited, but preferably from 1 to 4, and more preferably from 2 to 4 from the viewpoint of suppressing the content reduction of the compound represented by the formula (1).

The "aliphatic carboxylate" may be a carboxylic acid having an amino group as a substituent (amino acid), and a carboxylic acid having a hydroxy group as a substituent (oxy carboxylic acid) - In this case, the number of the substituent is preferably from 1 to 3, and particularly preferably from 1 to 2 from the viewpoint of suppressing the content reduction of the compound represented by the formula (1).

Specific examples of such aliphatic carboxylate include aspartic acid or a salt thereof or a solvate thereof such as sodium L-aspartate, potassium L-aspartate or magnesium L-aspartate; alanine or a salt thereof or a solvate thereof such as DL-alanine or L-alanine; arginine or a salt thereof or a solvate thereof such as L-arginine or L-arginine hydrochloride; epsilon-aminocaproic acid; edetic acid or a salt thereof or a solvate thereof such as calcium disodium edetate, disodium edetate hydrate, or tetrasodium edetate; citric acid or a salt thereof or a solvate thereof such as calcium citrate, citric acid hydrate, sodium citrate hydrate (trisodium citrate dihydrate), monobasic sodium citrate, dibasic sodium citrate,

anhydrous citric acid, or sodium citrate anhydrous; glycine or a salt thereof or a solvate thereof; gluconic acid or a salt thereof or a solvate thereof such as gluconic acid, calcium gluconate hydrate, sodium gluconate, or magnesium gluconate; glutamine or a salt thereof or a solvate thereof; glutamic acid or a salt thereof or a solvate thereof such as L-glutamic acid, L-glutamic acid hydrochloride, potassium L-glutamate, or sodium L-glutamate; succinic acid or a salt thereof or a solvate thereof such as succinic acid, monosodium succinate, or disodium succinate hexahydrate; acetic acid or a salt thereof or a solvate thereof such as acetic acid, ammonium acetate, potassium acetate, calcium acetate, sodium acetate hydrate, glacial acetic acid, or anhydrous sodium acetate; cysteine or a salt thereof or a solvate thereof such as L-cystine, L-cysteine, or L-cysteine hydrochloride hydrate; tartaric acid or a salt thereof or a solvate thereof such as tartaric acid, D-tartaric acid, potassium hydrogen tartrate, sodium DL-tartrate, sodium L-tartrate, or sodium potassium tartrate; sorbic acid or a salt thereof or a solvate thereof such as sorbic acid or potassium sorbate; lactic acid or a salt thereof or a solvate thereof such as lactic acid, sodium lactate solution, calcium lactate hydrate or aluminum lactate; histidine or a salt thereof or a solvate thereof such as L-histidine or L-histidine hydrochloride hydrate; phenylalanine or a salt thereof or a solvate thereof; malonic acid or a salt thereof or a solvate thereof; methionine or a salt thereof or a solvate thereof such as DL-methionine or L-methionine; lysine or a salt thereof or a solvate thereof such as L-lysine or L-lysine hydrochloride; malic acid or a salt thereof or a solvate thereof such as malic acid, DL-malic acid or sodium DL-malate; and leucine such as dl-leucine or L-leucine or a salt thereof or a solvate thereof. The aliphatic carboxylate is preferably an aliphatic carboxylic acid having 2 to 4 carboxyl groups or a salt thereof or a solvate thereof, and particulally preferably edetic acid, citric acid, tartaric acid, a salt thereof, or a solvate thereof.

All these aliphatic carboxylic acids are known compounds, so that they may be prepared by a known method or their commercial products may be used. Further, the aliphatic carboxylate in the form of a salt or a complex with another component may be used

The content of the acid in the aqueous composition is not particularly limited and can be appropriately determined, but it is preferably from 0.0001 to 4 w/v%, more preferably from 0.001 to 3 w/v%, and particularly preferably from 0.002 to 2.5 w/v% relative to the total volume of the aqueous composition, from the viewpoint of suppressing the content reduction of the compound represented by the formula (1). Particularly, in the case of using a borate as the acid, the content of the borate is not particularly limited and can be appropriately determined, but preferably from 0.003 to 2 w/v%, more preferably from 0.01 to 1 w/v%, and particularly preferably from 0.03 to 0.5 w/v% relative to the total volume of the aqueous composition, from the viewpoint of suppressing the content reduction of the compound represented by the formula (1). Further, when aliphatic carboxylate are used as the acid, the content of the aliphatic carboxylate is not particularly limited and can be appropriately determined, but from the viewpoint of suppressing the content reduction of the compound represented by the formula (1), it is preferably from 0.0001 to 3.5 w/v%, more preferably from 0.005 to 2.5 w/v%, and particularly preferably from 0.01 to 1 w/v% relative to the total volume of the aqueous composition.

The content mass ratio between the compound represented by the formula (1) or a salt thereof or a solvate thereof and the acid in the aqueous composition is not particularly limited, but, from the viewpoint of suppressing the content reduction of the compound represented by the formula (1), the content of the acid is preferably from 0.0003 to 200 parts by mass, more preferably from 0.003 to 80 parts by mass, and particularly preferably from 0.02 to 30 parts by mass relative to 1 part by mass of the compound represented by the formula (1) or a salt thereof or a solvate thereof in terms of a free form. Above all, in the case of using a borate as the acid, the content mass ratio of the borate is not particularly limited, but, from the viewpoint of suppressing the content reduction of the compound represented by the formula (1), the content of the borate is preferably from 0.0005 to 150 parts by mass, more preferably from 0.005 to 75 parts by mass, and particularly preferably from 0.03 to 25 parts by mass relative to 1 part by mass of the compound represented by the formula (1) or a salt thereof or a solvate thereof in terms of a free form. Further, in the case of using an aliphatic carboxylate as the acid, the content mass ratio of the aliphatic carboxylate is not particularly limited, but from the viewpoint of suppressing the content reduction of the compound represented by the formula (1), the content of the aliphatic carboxylate is preferably from 0.05 to 150 parts by mass, more preferably from 0.1 to 100 parts by mass, and particularly preferably from 0.3 to 75 parts by mass relative to 1 part by mass of the compound represented by the formula (1) or a salt thereof or a solvate thereof in terms of a free form.

In particular, in the case of using netarsudil as the compound represented by the formula (1) and using a borate as the acid, the content of the borate is preferably from 0.5 to 100 parts by mass, more preferably from 1 to 50 parts by mass, and particularly preferably from 2 to 20 parts by mass relative to 1 part by mass of netarsudil or a salt thereof or a solvate thereof in terms of a free form, from the viewpoint of suppressing the content reduction of netarsudil.

Further, in the case of using verosudil as the compound represented by the formula (1) and using a borate as the acid, the content of the borate is preferably from 0.001 to 5 parts by mass, more preferably from 0.01 to 1 part by mass, and particularly preferably from 0.05 to 0-5 parts by mass relative to 1 part by mass of verosudil or a salt thereof or a solvate thereof in terms of a free form, from the viewpoint of suppressing the content reduction of verosudil.

Furthermore, in the case of using the compound represented by the formula (8) as the compound represented by the formula (1) and using a borate as the acid, the content of the borate is preferably from 0.01 to 80 parts by mass, more preferably from 0.05 to 40 parts by mass, and particularly preferably from 0-1 to 20 parts by mass relative to 1 part

by mass of the compound represented by the formula (8) or a salt thereof or a solvate thereof in terms of a free form, from the viewpoint of suppressing the content reduction of the compound represented by the formula (8).

Furthermore, in the case of using netarsudil as the compound represented by the formula (1) and using an aliphatic carboxylate as the acid, the content of the aliphatic carboxylate is preferably from 0.05 to 180 parts by mass, more preferably from 0.1 to 130 parts by mass, and particularly preferably from 0-3 to 80 parts by mass relative to 1 part by mass of netarsudil or a salt thereof or a solvate thereof in terms of a free form, from the viewpoint of suppressing the content reduction of netarsudil.

Further, in the case of using verosudil as the compound represented by the formula (1) and using an aliphatic carboxylate as the acid, the content of the aliphatic carboxylate is preferably from 0.01 to 30 parts by mass, more preferably from 0.03 to 20 parts by mass, and particularly preferably from 0.5 to 10 parts by mass relative to 1 part by mass of verosudil or a salt thereof or a solvate thereof in terms of a free form, from the viewpoint of suppressing the content reduction of verosudil.

Furthermore, in the case of using the compound represented by the formula (8) as the compound represented by the formula (1) and using an aliphatic carboxylate as the acid, the content of the aliphatic carboxylate is preferably from 0.1 to 180 parts by mass, more preferably from 1 to 130 parts by mass, and particularly preferably from 3 to 80 parts by mass relative to 1 part by mass of the compound represented by the formula (8) or a salt thereof or a solvate thereof in terms of a free form, from the viewpoint of suppressing the content reduction of the compound represented by the formula (8).

In the specification, examples of the "quaternary ammonium type surfactant" include benzalkonium chloride, benzethonium chloride, and benzododecinium bromide, those are known as antiseptic and may be used singly or in combination- The quaternary ammonium type surfactant is preferably benzalkonium chloride or benzododecinium bromide, and particularly preferably benzalkonium chloride, from the viewpoint of suppressing the content reduction of the compound represented by the formula (1).

These quaternary ammonium type surfactants are known compounds, so that they may be prepared by a known method or their commercial products may be used

The content of the quaternary ammonium type surfactant in the aqueous composition is not particularly limited and may be appropriately determined, but, from the viewpoint of suppressing the content reduction of the compound represented by the formula (1), it is preferably from 0.00005 to 0.3 w/v%, more preferably from 0.00025 to 0.2 w/v%, and particularly preferably from 0.00025 to 0.15 w/v% relative to the total volume of the aqueous composition. Above all, in the case of using benzalkonium chloride as the quaternary ammonium type surfactant, the content of the benzalkonium chloride is preferably from 0.0001 to 0.3 w/v%, more preferably from 0.0005 to 0.2 w/v%, and particularly preferably from 0.0005 to 0.15 w/v% relative to the total volume of the aqueous composition, from the viewpoint of suppressing the content reduction of the compound represented by the formula (1). Further, in the case of using benzododecinium bromide as the quaternary ammonium type surfactant, the content of the benzododecinium bromide is preferably from 0.0001 to 0.15 w/v%, more preferably from 0.0005 to 0.05 w/v%, and particularly preferably from 0.0005 to 0.02 w/v% relative to the total volume of the aqueous composition, from the viewpoint of suppressing the content reduction of the compound represented by the formula (1).

The content mass ratio between the compound represented by the formula (1) or a salt thereof or a solvate thereof in the aqueous composition and the quaternary ammonium type surfactant is not particularly limited, but the content of the quaternary ammonium type surfactant is preferably from 0.0005 to 8 parts by mass, more preferably from 0.005 to 7 parts by mass, and particularly preferably from 0.007 to 6 parts by mass relative to 1 part by mass of the compound represented by the formula (1) or a salt thereof or a solvate thereof in terms of a free form, from the viewpoint of suppressing the content reduction of the compound represented by the formula (1).

In particular, in the case of using netarsudil as the compound represented by the formula (1) and using benzalkonium chloride as the quaternary ammonium type surfactant, the content of the benzalkonium chloride is preferably from 0.05 to 5 parts by mass, more preferably from 0.1 to 4 parts by mass, and particularly preferably from 0.5 to 3 parts by mass relative to 1 part by mass of netarsudil or a salt thereof or a solvate thereof in terms of a free form, from the viewpoint of suppressing the content reduction of netarsudil.

Further, in the case of using verosudil as the compound represented by the formula (1) and using benzalkonium chloride as the quaternary ammonium type surfactant, the content of the benzalkonium chloride is preferably from 0.001 to 1 part by mass, more preferably from 0.005 to 0.5 parts by mass, and particularly preferably from 0.01 to 0.3 parts by mass relative to 1 part by mass of verosudil or a salt thereof or a solvate thereof in terms of a free form, from the viewpoint of suppressing the content reduction of verosudil.

Furthermore, in the case of using a compound represented by the formula (8) as the compound represented by the formula (1) and using benzalkonium chloride as the quaternary ammonium type surfactant, the content of the benzalkonium chloride is preferably from 0.05 to 5 parts by mass, more preferably from 0.1 to 4 parts by mass, and particularly preferably from 0.5 to 3 parts by mass relative to 1 part by mass of the compound represented by the formula (8) or a salt thereof or a solvate thereof in terms of a free form, from the viewpoint of suppressing the content reduction

of the compound represented by the formula (8).

In the case of using netarsudil as the compound represented by the formula (1) and using benzododecinium bromide as the quaternary ammonium type surfactant, the content of the benzododecinium bromide is preferably from 0.07 to 5 parts by mass, more preferably from 0.2 to 4 parts by mass, and particularly preferably from 0.6 to 3 parts by mass relative to 1 part by mass of netarsudil or a salt thereof or a solvate thereof in terms of a free form, from the viewpoint of suppressing the content reduction of netarsudil.

Further, in the case of using verosudil as the compound represented by the formula (1) and using benzododecinium bromide as the quaternary ammonium type surfactant, the content of the benzododecinium bromide is preferably from 0.003 to 0.8 part by mass, more preferably from 0.007 to 0.4 parts by mass, and particularly preferably from 0.01 to 0.1 parts by mass relative to 1 part by mass of verosudil or a salt thereof or a solvate thereof in terms of a free form, from the viewpoint of suppressing the content reduction of verosudil.

Furthermore, in the case of using a compound represented by the formula (8) as the compound represented by the formula (1) and using benzododecinium bromide as the quaternary ammonium type surfactant, the content of the benzododecinium bromide is preferably from 0.07 to 5 parts by mass, more preferably from 0.2 to 4 parts by mass, and particularly preferably from 0.4 to 3 parts by mass relative to 1 part by mass of a compound represented by the formula (8) or a salt thereof or a solvate thereof in terms of a free form, from the viewpoint of suppressing the content reduction of the compound represented by the formula (8).

In the specification, the "polyhydric alcohol" means an alcohol having two or more hydroxy groups in the same molecule, and examples of the polyhydric alcohol include glycerin; saccharide such as fructose or glucose; sugar alcohol such as sorbitol, mannitol or xylitol; trometamol; propylene glycol; and macrogol (polyethylene glycol) such as macrogol 100, macrogol 200, macrogol 300, macrogol 400, macrogol 600, macrogol 1000, macrogol 1500, macrogol 1540, macrogol 4000, macrogol 6000, macrogol 20000 or macrogol 35000. These may be used singly or in combination- Among them, the polyhydric alcohol is preferably glycerin, sorbitol, propylene glycol, mannitol, or macrogol (among macrogols, it is preferable to use those having an average molecular weight of from 100 to 10000, more preferably from 200 to 8000. Incidentally, the average molecular weight of macrogol may be measured by the "Average Molecular Weight Test" described in the Article "macrogol 400" of Japanese Pharmacopoeia 17th Edition), and particularly preferably D-mannitol, macrogol 400, macrogol 4000 or macrogol 6000, from the viewpoint of suppressing the content reduction of the compound represented by the formula (1).

The polyhydric alcohols are known compounds, and they may be prepared by a known method or their commercial products may be used

The content of the polyhydric alcohol in the aqueous composition is not particularly limited and can be appropriately determined, but, from the viewpoint of suppressing the content reduction of the compound represented by the formula (1), it is preferably from 0.01 to 30 w/v%, more preferably from 0.1 to 20 w/v%, and particularly preferably from 0.5 to 5 w/v% relative to the total volume of the aqueous composition. Among all cases, in the case of using mannitol as the polyhydric alcohol, the content of mannitol is preferably from 0.1 to 25 w/v%, more preferably from 1 to 15 w/v%, and particularly preferably from 3 to 10 w/v% relative to the total volume of the aqueous composition, from the viewpoint of suppressing the content reduction of the compound represented by the formula (1). Additionally, in the case of using macrogol as the polyhydric alcohol, the content of macrogol is preferably from 0.01 to 20 w/v%, more preferably from 0.5 to 7 w/v%, and particularly preferably from 1 to 3 w/v% relative to the total volume of the aqueous composition, from the viewpoint of suppressing the content reduction of the compound represented by the formula (1).

The content mass ratio between the compound represented by the formula (1) or a salt thereof or a solvate thereof and the polyhydric alcohol in the aqueous composition is not particularly limited, but the content of the polyhydric alcohol is preferably from 0.01 to 2500 parts by mass, more preferably from 0.05 to 1500 parts by mass, and particularly preferably from 0.5 to 750 parts by mass relative to 1 part by mass of the compound represented by the formula (1) or a salt thereof or a solvate thereof in terms of a free form, from the viewpoint of suppressing the content reduction of the compound represented by the formula (1).

In particular, in the case of using netarsudil as the compound represented by the formula (1) and using mannitol as the polyhydric alcohol, the content of mannitol is preferably from 10 to 2000 parts by mass, more preferably from 20 to 1000 parts by mass, and particularly preferably from 30 to 500 parts by mass relative to 1 part by mass of netarsudil or a salt thereof or a solvate thereof in terms of a free form, from the viewpoint of suppressing the content reduction of netarsudil.

Further, in the case of using verosudil as the compound represented by the formula (1) and using mannitol as the polyhydric alcohol, the content of mannitol is preferably from 0.1 to 200 parts by mass, more preferably from 0.5 to 100 parts by mass, and particularly preferably from 1 to 50 parts by mass relative to 1 part by mass of verosudil or a salt thereof or a solvate thereof in terms of a free form, from the viewpoint of suppressing the content reduction of verosudil.

Furthermore, in the case of using a compound represented by the formula (8) as the compound represented by the formula (1) and using mannitol as the polyhydric alcohol, the content of mannitol is preferably from 10 to 2000 parts by mass, more preferably from 20 to 1000 parts by mass, and particularly preferably from 30 to 500 parts by mass

relative to 1 part by mass of the compound represented by the formula (8) or a salt thereof or a solvate thereof in terms of a free form, from the viewpoint of suppressing the content reduction of the compound represented by the formula (8).

Further, in the case of using netarsudil as the compound represented by the formula (1) and using macrogol as the polyhydric alcohol, the content of macrogol is preferably from 10 to 900 parts by mass, more preferably from 30 to 600 parts by mass, and particularly preferably from 50 to 300 parts by mass relative to 1 part by mass of netarsudil or a salt thereof or a solvate thereof in terms of a free form, from the viewpoint of suppressing the content reduction of netarsudil.

Further, in the case of using verosudil as the compound represented by the formula (1) and using macrogol as the polyhydric alcohol, the content of macrogol is preferably from 0.1 to 40 parts by mass, more preferably from 0.5 to 30 parts by mass, and particularly preferably from 1 to 20 parts by mass relative to 1 part by mass of verosudil or a salt thereof or a solvate thereof in terms of a free form, from the viewpoint of suppressing the content reduction of verosudil.

Furthermore, in the case of using a compound represented by the formula (8) as the compound represented by the formula (1) and using macrogol as the polyhydric alcohol, the content of macrogol is preferably from 10 to 900 parts by mass, more preferably from 30 to 600 parts by mass, and particularly preferably from 50 to 300 parts by mass relative to 1 part by mass of the compound represented by the formula (8) or a salt thereof or a solvate thereof in terms of a free form, from the viewpoint of suppressing the content reduction of the compound represented by the formula (8).

In the specification, "prostaglandin compounds" means one or more selected from the group consisting of a prostaglandin, a derivative thereof and a salt thereof (e-g-, an alkali metal salt such as a sodium salt or a potassium salt; an alkaline earth metal salt such as a calcium salt; and an ammonium salt) and a solvate thereof (e-g-, a hydrate). Specific examples of the prostaglandin compounds include isopropyl unoprostone (chemical name: (+)-isopropyl (Z)-7-[(1R,2R,3R,5S)-3,5-dihydroxy-2-(3-oxodecyl)cyclopentyl]hept-5-enoate), tafluprost (chemical name: 1-Methylethyl (5Z)-7-[(1R,2R,3R,5S)-2-[(1E) -3, 3-difluoro-4-phenoxy-1-butenyl]-3,5-dihydroxycyclopentyl]-5-heptenoate], traboprost (chemical name: Isopropyl (5Z) -7-((1R,2R,3R,5S)-3,5-dihydroxy-2-[(1E,3R)-3-hydroxy-4-[3-(trifluoromethyl)phenoxy]but-1-enyl]cyclopentyl)hept-5-enoate), bimatoprost (chemical name: (5Z) -7-{(1R,2R,3R,5S)-3,5-Dihydroxy-2-[(1E,3S)-3-hydroxy-5-phenylpent-1-en-1-yl]cyclopentyl}-N-ethylhept-5-enamide), latanoprost (chemical name: (+)-Isopropyl (Z) -7-[(1R,2R,3R,5S)-3,5-dihydroxy-2-[(3R)-3-hydroxy-5-phenylpentyl]cyclopentyl]-5-heptenoate) and its pharmaceutically acceptable salts, as well as solvates of these compounds and their pharmaceutically acceptable salts with water, alcohols and the like. These may be used singly or in combination.

As the prostaglandin compounds, one or more selected from the group consisting of tafluprost, travoprost, bimatoprost, latanoprost, a salt thereof, and a solvate thereof are preferable, and latanoprost is particularly preferable, from the viewpoint of suppressing the content reduction of the compound represented by the general formula (1).

All these prostaglandin compounds are known compounds, and they may be produced by a known method, or their commercially available products may be used

The content of the prostaglandin in the aqueous composition is not particularly limited and may be appropriately determined, but, from the viewpoint of suppressing the content reduction of the compound represented by the formula (1), it is preferably from 0.00005 to 3 w/v%, more preferably from 0.00025 to 0.25 w/v%, and particularly preferably from 0.00075 to 0.075 w/v% relative to the total volume of the aqueous composition. Above all, in the case of using latanoprost or a salt thereof or a solvate thereof as the prostaglandin compound, the content of the same is preferably from 0.0001 to 0.1 w/v%, more preferably from 0.0005 to 0.05 w/v%, and particularly preferably from 0.001 to 0.01 w/v% relative to the total volume of the aqueous composition in terms of a free form of latanoprost, from the viewpoint of suppressing the content reduction of the compound represented by the formula (1) Further, in the case of using tafluprost or a salt thereof or a solvate thereof as the prostaglandin compound, the content of the same is preferably from 0.0001 to 0.02 w/v%, more preferably from 0.0005 to 0.01 w/v%, and particularly preferably from 0.001 to 0.005 w/v% relative to the total volume of the aqueous composition in terms of a free form of tafluprost, from the viewpoint of suppressing the content reduction of the compound represented by the formula (1). Further, in the case of using travoprost or a salt thereof or a solvate thereof as the prostaglandin compound, the content of the same is preferably from 0.0001 to 0.05 w/v%, more preferably from 0.0005 to 0.01 w/v%, and particularly preferably from 0.001 to 0.005 w/v% relative to the total volume of the aqueous composition in terms of a free form of travoprost, from the viewpoint of suppressing the content reduction of the compound represented by the formula (1). Further, in the case of using bimatoprost or a salt thereof or a solvate thereof as the prostaglandin compound, the content of the same is preferably from 0.001 to 2 w/v%, more preferably from 0.005 to 1 w/v%, and particularly preferably from 0.01 to 0.5 w/v% relative to the total volume of the aqueous composition in terms of a free form of bimatoprost, from the viewpoint of suppressing the content reduction of the compound represented by the formula (1).

The content mass ratio between the compound represented by the formula (1) or a salt thereof or a solvate thereof and the prostaglandin compound in the aqueous composition is not particularly limited, but, from the viewpoint of suppressing the content reduction of the compound represented by the formula (1), the content of the prostaglandin compound is preferably from 0.0001 to 50 parts by mass, more preferably from 0.0125 to 12.5 parts by mass, and particularly preferably from 0.0375 to 3.75 parts by mass in terms of a free form, relative to 1 part by mass of the

compound represented by the formula (1) or a salt thereof or a solvate thereof in terms of a free form.

In particular, in the case of using netarsudil as the compound represented by the formula (1) and using latanoprost or a salt thereof or a solvate thereof as the prostaglandin compound, the content of latanoprost is preferably from 0.005 to 5 parts by mass, more preferably from 0.025 to 2.5 parts by mass, and particularly preferably from 0.05 to 0.5 parts by mass in terms of a free form, relative to 1 part by mass of netarsudil or a salt thereof or a solvate thereof in terms of a free form, from the viewpoint of suppressing the content reduction of netarsudil. Further, in the case of using netarsudil as the compound represented by the formula (1) and using tafluprost or a salt thereof or a solvate thereof as the prostaglandin compound, the content of tafluprost is preferably from 0.005 to 1 parts by mass, more preferably from 0.025 to 0.5 parts by mass, and particularly preferably from 0.05 to 0.25 parts by mass in terms of a free form, relative to 1 part by mass of netarsudil or a salt thereof or a solvate thereof in terms of a free form, from the viewpoint of suppressing the content reduction of netarsudil. Further, in the case of using netarsudil as the compound represented by the formula (1) and using travoprost or a salt thereof or a solvate thereof as the prostaglandin compound, the content of travoprost is preferably from 0.005 to 2.5 parts by mass, more preferably from 0.025 to 0.5 parts by mass, and particularly preferably from 0.05 to 0.25 parts by mass in terms of a free form, relative to 1 part by mass of netarsudil or a salt thereof or a solvate thereof in terms of a free form, from the viewpoint of suppressing the content reduction of netarsudil. Further, in the case of using netarsudil as the compound represented by the formula (1) and using bimatoprost or a salt thereof or a solvate thereof as the prostaglandin compound, the content of bimatoprost is preferably from 1 to 30 parts by mass, more preferably from 5 to 25 parts by mass, and particularly preferably from 10 to 20 parts by mass in terms of a free form, relative to 1 part by mass of netarsudil or a salt thereof or a solvate thereof in terms of a free form, from the viewpoint of suppressing the content reduction of netarsudil.

In addition, in the case of using verosudil as the compound represented by the formula (1) and using latanoprost or a salt thereof or a solvate thereof as the prostaglandin compound, the content of latanoprost is preferably from 0.001 to 2 parts by mass, more preferably from 0.003 to 1 parts by mass, and particularly preferably from 0.005 to 0.2 parts by mass in terms of a free form, relative to 1 part by mass of verosudil or a salt thereof or a solvate thereof in terms of a free form, from the viewpoint of suppressing the content reduction of verosudil. Further, in the case of using verosudil as the compound represented by the formula (1) and using tafluprost or a salt thereof or a solvate thereof as the prostaglandin compound, the content of tafluprost is preferably from 0.0005 to 3 parts by mass, more preferably from 0.001 to 0.2 parts by mass, and particularly preferably from 0.002 to 0.1 parts by mass in terms of a free form, relative to 1 part by mass of verosudil or a salt thereof or a solvate thereof in terms of a free form, from the viewpoint of suppressing the content reduction of verosudil. Further, in the case of using verosudil as the compound represented by the formula (1) and using travoprost or a salt thereof or a solvate thereof as the prostaglandin compound, the content of travoprost is preferably from 0.0003 to 0.2 parts by mass, more preferably from 0.001 to 0.1 parts by mass, and particularly preferably from 0.005 to 0.05 parts by mass in terms of a free form, relative to 1 part by mass of verosudil or a salt thereof or a solvate thereof in terms of a free form, from the viewpoint of suppressing the content reduction of verosudil. Further, in the case of using verosudil as the compound represented by the formula (1) and using bimatoprost or a salt thereof or a solvate thereof as the prostaglandin compound, the content of bimatoprost is preferably from 0.01 to 10 parts by mass, more preferably from 0.05 to 5 parts by mass, and particularly preferably from 0.1 to 2 parts by mass in terms of a free form, relative to 1 part by mass of verosudil or a salt thereof or a solvate thereof in terms of a free form, from the viewpoint of suppressing the content reduction of verosudil.

In addition, in the case of using a compound represented by the formula (8) as the compound represented by the formula (1) and using latanoprost or a salt thereof or a solvate thereof as the prostaglandin compound, the content of latanoprost is preferably from 0.005 to 5 parts by mass, more preferably from 0.025 to 2.5 parts by mass, and particularly preferably from 0.05 to 0.5 parts by mass in terms of a free form, relative to 1 part by mass of the compound represented by the formula (8) or a salt thereof or a solvate thereof in terms of a free form, from the viewpoint of suppressing the content reduction of the compound represented by the formula (8). Further, in the case of using a compound represented by the formula (8) as the compound represented by the formula (1) and using tafluprost or a salt thereof or a solvate thereof as the prostaglandin compound, the content of tafluprost is preferably from 0.005 to 1 parts by mass, more preferably from 0.025 to 0.5 parts by mass, and particularly preferably from 0.05 to 0.25 parts by mass in terms of a free form, relative to 1 part by mass of the compound represented by the formula (8) or a salt thereof or a solvate thereof in terms of a free form, from the viewpoint of suppressing the content reduction of the compound represented by the formula (8). Further, in the case of using a compound represented by the formula (8) as the compound represented by the formula (1) and using travoprost or a salt thereof or a solvate thereof as the prostaglandin compound, the content of travoprost is preferably from 0.005 to 2.5 parts by mass, more preferably from 0.025 to 0.5 parts by mass, and particularly preferably from 0.05 to 0.25 parts by mass in terms of a free form, relative to 1 part by mass of the compound represented by the formula (8) or a salt thereof or a solvate thereof in terms of a free form, from the viewpoint of suppressing the content reduction of the compound represented by the formula (8). Further, in the case of using a compound represented by the formula (8) as the compound represented by the formula (1) and using bimatoprost or a salt thereof or a solvate thereof as the prostaglandin compound, the content of bimatoprost is preferably from 1 to 30 parts by mass, more

preferably from 5 to 25 parts by mass, and particularly preferably from 10 to 20 parts by mass in terms of a free form, relative to 1 part by mass of the compound represented by the formula (8) or a salt thereof or a solvate thereof in terms of a free form, from the viewpoint of suppressing the content reduction of the compound represented by the formula (8).

In the present specification, the "aqueous composition" means a composition comprising at least water, and the property of the aqueous composition is not particularly limited as long as it can be housed in a container described below, and for example, the aqueous composition may be in the form of liquid (solution or suspension), semi-solid (ointment), and the like. Examples of the water in the composition include purified water, water for injection, and sterilized purified water.

The content of water in the aqueous composition is not particularly limited, but preferably 5 % by mass or more, more preferably 20 % by mass or more, still more preferably 50 % by mass or more, even more preferably 90 % by mass or more, and particularly preferably 90 to 99.99 % by mass.

The aqueous composition may comprise one or more additives utilized in pharmaceutical products, quasi-drugs and the like, besides the components described above, depending on the dosage form. Examples of the additives include an inorganic salt, an isotonic agent, a chelating agent, a stabilizing agent, a pH adjusting agent, a preservative, an antioxidant, a thickening agent, a surfactant, a solubilizer, a suspending agent, a refreshing agent, a dispersing agent, a preserving agent, an oily base, an emulsion base, and a water-soluble base.

Specific examples of the additive include sodium bisulfite, benzyl benzoate, fennel oil, ethanol, an ethylene-vinyl acetate copolymer, potassium chloride, calcium chloride hydrate, sodium chloride, magnesium chloride, alkyldiaminoethylglycine hydrochloride solution, a carboxyvinyl polymer, dried sodium sulfite, dried sodium carbonate, d-camphor, dl-camphor, creatinine, chlorobutanol, geraniol, sodium chondroitin sulfate, titanium oxide, dibutylhydroxytoluene, potassium bromide, sodium hydroxide, polyoxyl 45 stearate, purified lanolin, taurine, sodium hydrogen carbonate, sodium carbonate hydrate, sodium thiosulfate hydrate, tyloxapol, sodium dehydroacetate, a concentrated mixed tocopherol, white petrolatum, peppermint water, peppermint oil, ethyl parahydroxybenzoate, butyl parahydroxybenzoate, propyl parahydroxybenzoate, methyl parahydroxybenzoate, human serum albumin, sodium pyrosulfite, phenyl ethyl alcohol, bergamot oil, benzyl alcohol, povidone, polyoxyethylene (200) polyoxypropylene glycol (70), sodium polystyrenesulfonate, polysorbate 80, polyoxyethylene hydrogenated castor oil 60, d-borneol, methanesulfonic acid, l-menthol, monoethanolamine, aluminum monostearate, polyethylene glycol monostearate, eucalyptus oil, potassium iodide, oxyquinoline sulfate, liquid paraffin, borneol, and vaseline. These may be used singly or in combination.

Among these additives, it is preferable to use potassium chloride, calcium chloride hydrate, sodium chloride, magnesium chloride, sodium hydroxide, sodium hydrogen carbonate, sodium carbonate hydrate, povidone, polysorbate 80, polyoxyethylene hydrogenated castor oil, polyethylene glycol monostearate, monoethanolamine, l-menthol or the like.

The aqueous composition may further comprise, in addition to the components described above, one or more other medicinal components, depending on the applicable disease or the like. Examples of the medicinal components include α1 receptor blockers including bunazosin or a salt thereof or a solvate thereof such as bunazosin hydrochloride; α2 receptor agonists including brimonidine or a salt thereof or a solvate thereof such as brimonidine tartrate, and apraclonidine or a salt thereof or a solvate thereof; β-blockers including carteolol or a salt thereof or a solvate thereof such as carteolol hydrochloride, nipradilol or a salt thereof or a solvate thereof, timolol or a salt thereof or a solvate thereof such as timolol maleate, betaxolol or a salt thereof or a solvate thereof such as betaxolol hydrochloride, levobunolol or a salt thereof or a solvate thereof such as levobunolol hydrochloride, befunolol or a salt thereof or a solvate thereof, and metipranolol or a salt thereof or a solvate thereof; carbonic anhydrase inhibitors including dorzolamide or a salt thereof or a solvate thereof such as dorzolamide hydrochloride, brinzolamide or a salt thereof or a solvate thereof, acetazolamide or a salt thereof or a solvate thereof, dichlorphenamide or a salt thereof or a solvate thereof, and methazolamide or a salt thereof or a solvate thereof; Rho kinase inhibitors including Ripasudil or a salt thereof or a solvate thereof, Y-39983, and H-1129; sympathomimetic drugs including dipivefrine or a salt thereof or a solvate thereof such as dipivefrin hydrochloride, and epinephrine or a salt thereof or a solvate thereof such as epinephrine, epinephrine borate, or epinephrine hydrochloride; parasympathomimetic drugs including distigmine bromide or a salt thereof or a solvate thereof, pilocarpine or a salt thereof or a solvate thereof such as pilocarpine, pilocarpine hydrochloride or pilocarpine nitrate, and carbachol or a salt thereof or a solvate thereof; calcium antagonists including lomerizine or a salt thereof or a solvate thereof such as lomerizine hydrochloride; and cholinesterase inhibitors including demecarium or a salt thereof or a solvate thereof, echothiophate or a salt thereof or a solvate thereof, and physostigmine or a salt thereof or a solvate thereof. These medicinal components can be blended singly or a combination of two or more

As the other medicinal components, β-blockers are preferred, and especially timolol is preferred.

When one or more selected from the group consisting of the acid, the quaternary ammonium type surfactant and the polyhydric alcohol are contained as the component (B), the aqueous composition may not contain the above-mentioned prostaglandin compounds.

One embodiment of the aqueous compositions used in the present invention includes, for example, those other than the following <A-1> to <A-10>:

<A-1> a composition comprising (rac) -2- (dimethylamino) -N-(1-hydroxy-isoquinolin-6-yl) -2- (thiophen-3-yl) acetamide hydrochloride, travoprost, boric acid, D-mannitol, benzalkonium chloride, polyoxyl 40 stearate, polyethylene glycol 400, EDTA, and purified water.
<A-2> a composition comprising (rac) -2- (dimethylamino) -N-(1-hydroxy-isoquinolin-6-yl) -2- (thiophen-3-yl) acetamide hydrochloride, travoprost, boric acid, D-mannitol, benzalkonium chloride, Cremophor RH40, polyethylene glycol 400, EDTA, and purified water.
<A-3> a composition comprising (rac) -2- (dimethylamino) -N-(1-hydroxy-isoquinolin-6-yl) -2- (thiophen-3-yl) acetamide hydrochloride, travoprost, boric acid, D-mannitol, polyoxyl 40 stearate, polyethylene glycol 400, EDTA, and purified water.
<A-4> a composition comprising (rac) -2- (dimethylamino) -N-(1-hydroxy-isoquinolin-6-yl) -2- (thiophen-3-yl) acetamide hydrochloride, latanoprost, monobasic sodium phosphate, dibasic sodium phosphate, benzalkonium chloride, sodium chloride, EDTA, and purified water.
<A-5> a composition comprising (rac) -2- (dimethylamino) -N-(1-hydroxy-isoquinolin-6-yl) -2- (thiophen-3-yl) acetamide hydrochloride, latanoprost, boric acid, D-mannitol, benzalkonium chloride, EDTA, and purified water.
<A-6> a composition comprising (rac) -2- (dimethylamino) -N-(1-hydroxy-isoquinolin-6-yl)-2-(thiophen-3-yl)acetamide hydrochloride, bimatoprost, monobasic sodium phosphate, dibasic sodium phosphate, benzalkonium chloride, sodium chloride, EDTA, and purified water.
<A-7> a composition comprising (rac) -2- (dimethylamino) -N-(1-hydroxy-isoquinolin-6-yl) -2- (thiophen-3-yl) acetamide hydrochloride, bimatoprost, monobasic sodium phosphate, dibasic sodium phosphate, benzalkonium chloride, sodium chloride, EDTA, and purified water.
<A-8> a composition comprising (rac) -2- (dimethylamino) -N-(1-hydroxy-isoquinolin-6-yl) -2- (thiophen-3-yl) acetamide hydrochloride, bimatoprost, boric acid, D-mannitol, and purified water.
<A-9> a composition comprising (S)-4-(3-amino-1-(isoquinolin-6-ylamino)-1-oxopropan-2-yl)benzyl 2,4-dimethylbenzoate, travoprost, boric acid, D-mannitol, benzalkonium chloride, polyoxyl 40 stearate, polyethylene glycol 400, EDTA, and purified water.
<A-10> a composition comprising (S)-4-(3-amino-1-(isoquinolin-6-ylamino)-1-oxopropan-2-yl)benzyl 2,4-dimethylbenzoate, latanoprost, boric acid, D-mannitol, benzalkonium chloride, polyoxyl 40 stearate, polyethylene glycol 400, EDTA, and purified water.

The pH of the aqueous composition (at 25°C) is not particularly limited, but preferably from 3 to 9, more preferably from 3.5 to 8, still more preferably from 4 to 7, particularly preferably from 5 to 6. Further, the osmotic pressure ratio of the aqueous composition to physiological saline is not particularly limited, but preferably from 0.6 to 3, particularly preferably from 0.6 to 2.

The pharmaceutical preparation of the present invention uses a container formed of a polyolefin resin.

In the present specification, a "container" means a package for directly housing the aqueous composition. The concept of the container encompasses any of "well-closed container", "tight container" and "hermetic container" which are defined in the Japanese Pharmacopoeia, Seventeenth Edition, General Notices.

The form of the container is not particularly limited as long as it is capable of housing the aqueous composition, and may be appropriately selected and set depending on the dosage form, the application of the pharmaceutical preparation or the like. Specific examples of the container in such a form include a container for injection, a container for inhalation, a container for spray, a bottle-shaped container, a tube-shaped container, an eye drop container, a nasal drop container, an ear drop container, a bag shaped container and the like.

The container is preferably an eye drop container from the viewpoint of advantageously utilizing the pharmacological action of the compound represented by the formula (1).

In the specification, the "container formed of a polyolefin resin" means a container of which at least a part in contact with the aqueous composition is "formed of a polyolefin resin". Thus, for example, a container having a polyolefin resin layer as an inner layer in contact with an aqueous composition, and further having a layer of other resin material laminated on the outside of the inner layer also corresponds to the "container formed of a polyolefin resin". The polyolefin resin is not particularly limited, and it may be a polymer composed of single kind of monomer (homopolymer) or a copolymer composed of plural kinds of monomers (copolymer). In the case of a copolymer, the polymerization mode is not particularly limited, and the copolymer may be a random copolymer or a block copolymer. Furthermore, the stereoregularity (tacticity) of the copolymer is not particularly limited.

Specific examples of the polyolefin resin include polyethylene (more specifically, including, low density polyethylene (linear low density polyethylene), high density polyethylene, medium density polyethylene), polypropylene, cyclic polyolefin, poly(4-methylpentene), polytetrafluoroethylene, an ethylene-propylene copolymer, an ethylene-$\alpha$-olefin copolymer, an ethylene-acrylic acid copolymer, an ethylene-methacrylic acid copolymer, an ethylene-vinyl acetate copolymer, and an ethylene-ethyl acrylate copolymer. These resins can be used singly or in a combination of two or more. From the viewpoint of suppressing the content reduction of the compound represented by the formula (1) due to storage

under high temperature, the polyolefin resin is preferably, polyethylene, polypropylene, or cyclic polyolefin, more preferably, polyethylene or polypropylene.

As used herein, the phrase "formed of a polyolefin resin" means that the material comprises, at least in part, a polyolefin resin. For example, the one formed of a mixture of two or more kinds of resin (polymer alloy) of a polyolefin resin and one or more other kinds of resin also correspond to the one "formed of a polyolefin resin".

It is preferred that a substance which interferes with the transmission of ultraviolet light, such as an ultraviolet absorbing agent or an ultraviolet scattering agent is kneaded in a container. With the container in which such a substance has been kneaded, the light stability of the compound represented by the formula (1) is improved. Specific examples of the ultraviolet scattering agent include titanium oxide and zinc oxide. Examples of the ultraviolet absorbing agent include: benzotriazole-based ultraviolet absorbent such as 2- (2H-benzotriazol-2-yl) -p-cresol (e.g., Tinuvin P: BASF), 2-(2H-benzotriazol-2-yl)-4,6-bis(1-methyl-1-phenylethyl) phenol (e.g., Tinuvin 234: BASF), 2-(3,5-di-tert-butyl-2-hydroxyphenyl)benzotriazole (e.g., Tinuvin 320: BASF), 2-[5-chloro-(2H)-benzotriazol-2-yl]-4-methyl-6-t-butyl-phenol (e.g., Tinuvin 326: BASF), 2-(3,5-di-t-butyl-2-hydroxyphenyl)-5-chlorobenzotriazole (e.g., Tinuvin 327: BASF), 2-(2H-benzotriazol-2-yl)-4,6-di-tert-pentylphenol (e.g., Tinuvin PA328: BASF), 2- (2H-benzotriazol-2-yl)-4-(1,1,3,3-tetramethylbutyl) phenol (e.g., Tinuvin 329: BASF), 2,2'-methylenebis [6-(2H-benzotriazol-2-yl)-4-(1,1,3,3-tetramethylbutyl)phenol (e.g., Tinuvin 360: BASF), a reaction product of methyl 3-(3-(2H-benzotriazol-2-yl)-5-tert-butyl-4-hydroxyphenyl)propionate and polyethylene glycol 300 (e.g., Tinuvin 213: BASF), 2-(2H-benzotriazol-2-yl)-6-dodecyl-4-methylphenol (e.g., Tinuvin 571: BASF), 2-(2'-hydroxy-3' ,5'-di-t-amylphenyl)benzotriazole, 2-[2'-hydroxy-3'-(3",4",5",6"-tetrahydrophthalimidemethyl)-5'-methylphenyl]benzotriazole, and 2,2'-methylenebis[4-(1,1,3,3-tetramethylbutyl)-6-(2H-benzotriazol-2-yl)phenol]; cyanoacrylate-based ultraviolet absorbing agent such as 2,2-bis{[2-cyano-3,3-diphenyl acryloyloxy]methyl}propane-1,3-diyl=bis(2-cyano-3,3-diphenylacrylate) (for example, Uvinul 3030 FF: BASF), ethyl 2-cyano-3,3-diphenyl acrylate (e.g., Uvinul 3035: BASF), and 2-ethylhexyl 2-cyano-3,3-diphenylacrylate (e.g., Uvinul 3039: BASF) and others; triazine-based ultraviolet absorbing agent such as 2-(4,6-diphenyl-1,3,5-triazin-2-yl)-5-[(hexyl)oxy]-phenol (e.g., Tinuvin 1577 ED: BASF); benzophenone-based ultraviolet absorbing agent such as octabenzone (e.g., Chimassorb 81: BASF), 2,2'-dihydroxy-4,4'-dimethoxy benzophenone (e.g.,Uvinul 3049: BASF), 2,2'-4,4'-tetrahydrobenzophenone (e.g., Uvinul 3050: BASF), oxybenzone, hydroxymethoxybenzophenone sulfonic acid, sodium hydroxymethoxybenzophenone sulfonate, dihydroxydimethoxybenzophenone, sodium dihydroxydimethoxybenzophenone disulfonate, dihydroxybenzophenone, and tetrahydroxybenzophenone; cinnamic acid-based ultraviolet absorbing agent such as methyl diisopropylcinnamate, cinoxate, glyceryl mono-2-ethylhexanoate diparamethoxycinnamate, a mixture of isopropyl paramethoxycinnamate and diisopropyl cinnamate, 2-ethylhexyl paramethoxycinnamate, and benzyl cinnamate; benzoic acid ester-based ultraviolet absorbing agent such as para-aminobenzoic acid, ethyl para-aminobenzoate, glyceryl para-aminobenzoate, amyl para-dimethylaminobenzoate, 2-ethylhexyl para-dimethylaminobenzoate, and ethyl 4-[N,N-di(2-hydroxypropyl) amino]benzoate; salicylic acid-based ultraviolet absorbing agent such as ethylene glycol salicylate, octyl salicylate, dipropylene glycol salicylate, phenyl salicylate, homomenthyl salicylate, and methyl salicylate; guaiazulene; 2-ethylhexyl dimethoxybenzylidene dioxoimidazolidine propionate; 2,4,6-tris[4-(2-ethylhexyloxycarbonyl)anilino] 1,3,5-triazine; parahydroxy anisole; 4-tert-butyl-4'-methoxydibenzoylmethane; phenylbenzimidazole sulfonate; and hexyl 2-(4-diethylamino-2-hydroxybenzoyl) benzoate.

When a substance which interferes with the transmission of ultraviolet light is kneaded in the container, the blending ratio is determined depending on the kinds of the substance and the like, but, for example, the ratio of the substance in the container may be about from 0.001 to 50 % by mass, preferably from 0.002 to 25 % by mass, particularly preferably from 0.01 to 10 % by mass.

It is preferred that the inside of the container formed of a polyolefin resin is visible (observable) to the naked eye or the like. When the inside is visible, there are some advantages that it becomes possible to examine the presence or absence of contamination or the like in the manufacturing process of the pharmaceutical preparation; and that the user of the pharmaceutical preparation can check the remained content (aqueous composition), and the like. Here, it is sufficient if the visibility is ensured in at least a portion of the surface of the container (For example, an eye drop container having a bottom surface allowing the inside of the container to be visible corresponds to the container which is visible, even when the inside of the container is not visible from the side surface owing to a shrink film or the like) - When the inside of the container is visible from at least a portion of the surface of the container, it is possible to confirm the aqueous composition inside of the container.

The means for housing the aqueous composition in the container formed of a polyolefin resin is not particularly limited and it can be performed by filling or the like with an ordinary method depending on the form of the container or the like.

The pharmaceutical preparation or aqueous composition can be formulated in various dosage forms according to a known method described in the Japanese Pharmacopoeia, Seventeenth Edition, General Rules for Preparation or the like. Examples of the dosage form include injection, inhalation liquid, eye drop, eye ointment, ear drop, nasal drop liquid, enema formulation, topical liquid, spray, ointment, gel, oral liquid, and syrup. From the viewpoint of advantageously utilizing the pharmacological actions of the compound represented by the formula (1), the dosage form is preferably, a

dosage form for eye disease, specifically, eye drop or eye ointment, and particularly preferably, eye drop,

The applicable disease of the pharmaceutical preparation is not particularly limited, and it is appropriately selected depending on the pharmacological actions of the compound represented by the formula (1) or the like.

Specifically, for example, the pharmaceutical preparation can be used as a prevention or treatment agent for ocular hypertension and glaucoma, based on Rho kinase inhibitory activity, norepinephrine transporter inhibitory activity, and intraocular pressure-decreasing activity of the compound represented by the formula (1). Detailed examples of the glaucoma include primary open-angle glaucoma, normal tension glaucoma, hypersecretion glaucoma, acute angle closure glaucoma, chronic angle closure glaucoma, plateau iris syndrome, mixed-type glaucoma, steroid glaucoma, glaucoma capsulare, pigment glaucoma, amyloid glaucoma, angiogenesis glaucoma, and malignant glaucoma.

In the case of using the aqueous composition or pharmaceutical preparation as a prevention or treatment agent for eye disease (appropriately, a diseases selected from the group of ocular hypertension and glaucoma), the aqueous composition or pharmaceutical preparation may be administered, for example, about 1 to 3 times a day in a suitable dose.

Hereinafter, examples of embodiments or aspects of the present invention are disclosed below; however, the present invention is not limited thereto.

[1] A pharmaceutical preparation comprising an aqueous composition comprising the following components (A) and (B):

(A) a compound represented by the following formula (1)

(1)

(in the formula, $R_1$ and $R_2$ each independently represent a hydrogen atom or a $C_1$-$C_4$ alkyl group,
$R_3$ represents a hydrogen atom or a hydroxy group,
"A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and
the formula (1) also includes a tautomer thereof)
or a salt thereof or a solvate thereof; and

(B) one or more selected from the group consisting of an acid, a quaternary ammonium type surfactant, a polyhydric alcohol and a prostaglandin compound,
wherein the aqueous composition is housed in a container formed of a polyolefin resin.

[2] The pharmaceutical preparation according to [1], wherein the compound represented by the formula (1) is a compound represented by the following formula (2), (5), (5') or (8):

(2) ;

(5) ;

(5') ;

(8) .

[3] The pharmaceutical preparation according to [1], wherein the compound represented by the formula (1) is a compound represented by the following formula (3) or (6):

(3) ;

rac

(6) .

[4] The pharmaceutical preparation according to [1], wherein the compound represented by the formula (1) is a compound represented by the following formula (4) or (7):

(4) ;

(7) .

[5] The pharmaceutical preparation according to any one of [1] to [4], wherein the polyolefin resin is polyethylene or polypropylene.
[6] The pharmaceutical preparation according to any one of [1] to [5], wherein the acid is one or more selected from the group consisting of boric acid, aliphatic carboxylate, a salt thereof, and a solvate thereof.
[7] The pharmaceutical preparation according to any one of [1] to [6], wherein the quaternary ammonium type surfactant is one or more selected from the group consisting of benzalkonium chloride and benzododecinium bromide.
[8] The pharmaceutical preparation according to any one of [1] to [7], wherein the polyhydric alcohol is one or more selected from the group consisting of glycerin, sorbitol, propylene glycol, mannitol and macrogol.
[9] The pharmaceutical preparation according to any one of [1] to [8], wherein the prostaglandin compound is one or more selected from the group consisting of tafluprost, travoprost, bimatoprost, latanoprost, a salt thereof, and a solvate thereof.
[10] A method comprising the steps of:

preparing an aqueous composition comprising the compound represented by the formula (1) or a salt thereof or a solvate thereof;
incorporating one or more selected from the group consisting of an acid, a quaternary ammonium type surfactant, a polyhydric alcohol and a prostaglandin compound into the aqueous composition; and
housing the aqueous compound in a container formed of a polyolefin resin,
the method being for suppressing the content reduction (e.g. adsorption) of the compound represented by the formula (1) or a salt thereof or a solvate thereof in the aqueous composition.

[11] The method according to [10], wherein the compound represented by the formula (1) is a compound represented by the formula (2), (5), (5') or (8).
[12] The method according to [10], wherein the compound represented by the formula (1) is a compound represented by the formula (3) or (6).
[13] The method according to [10], wherein the compound represented by the formula (1) is a compound represented by the formula (4) or (7).
[14] The method according to any one of [10] to [13], wherein the polyolefin resin is polyethylene or polypropylene.
[15] The method according to [10] to [14], wherein the acid is one or more selected from the group consisting of boric acid, aliphatic carboxylate, a salt thereof, and a solvate thereof.
[16] The method according to any one of [10] to [15], wherein the quaternary ammonium type surfactant is one or more selected from the group consisting of benzalkonium chloride and benzododecinium bromide.
[17] The method according to any one of [10] to [16], wherein the polyhydric alcohol is one or more selected from the group consisting of glycerin, sorbitol, propylene glycol, mannitol and macrogol.
[18] The method according to any one of [10] to [17], wherein the prostaglandin compound is one or more selected from the group consisting of tafluprost, travoprost, bimatoprost, latanoprost, a salt thereof, and a solvate thereof.

EXAMPLES:

Next, the present invention is further described by way of Examples, but the present invention is not limited to these Examples.

In the following Test Examples, the measurements of netarsudil using HPLC were performed using an ODS column as the column, 0.01 mol/L phosphate buffer solution and acetonitrile as the mobile phase, and a UV absorptiometer as the detector (wavelength: 254 nm), respectively.

[Test Example 1] Adsorption Inhibition Test 1

To investigate the presence or absence of adsorption of netarsudil to the container formed of a polyolefin resin, a piece formed of a polyolefin resin was placed in an aqueous composition comprising netarsudil and stored for a certain period of time and then the presence or absence of the content reduction of netarsudil in the aqueous composition was examined.

In other words, aqueous compositions shown in Table 1 were prepared with an ordinary method, and 5 mL of the obtained composition each was housed in a glass container, and then, into the aqueous composition, three resin pieces (each having a size of 1 cm x 2 cm) formed of polypropylene (PP) were immersed to obtain a pharmaceutical preparation of Reference Example 1 or 2. In addition, a pharmaceutical preparation of Reference Example 3 was prepared in the same way as Reference Example 2 except for not immersing resin pieces formed of polypropylene.

Each of the obtained pharmaceutical preparations was stored at 60°C for a week. To examine the presence or absence of content reduction of netarsudil after storage, the concentrations of netarsudil in the aqueous composition before and after storage were measured for each of the pharmaceutical preparations. The concentration of netarsudil in the aqueous composition was calculated by measuring the ratio of the peak area of the aqueous composition relative to the peak area of a netarsudil solution of a known concentration, using HPLC.

Then, based on the calculated concentration of netarsudil in the aqueous composition, the residual rate (%) of netarsudil was assessed using the following expression:

Residual rate (%) = {(Concentration of netarsudil in the aqueous composition after storage)/(Concentration of netarsudil in the aqueous composition before storage)} × 100

The results are shown in Table 1.

[Table 1]

| | | Reference Example 1 | Reference Example 2 | Reference Example 3 |
|---|---|---|---|---|
| Aqueous composition (amount per 100 mL) | Netarsud dimesylate | 0.02848 g (002 g n terms of a free form) | 0.02848 g (002 g n terms of a free form) | 0.02848 g (002 g n terms of a free form) |
| | Boric acid | 0.05 g | - | - |
| | Sod um hydrox de | q.s. (pH 50) | q.s. (pH 50) | q.s. (pH 50) |
| | Purified water | Balance | Balance | Balance |
| Presence or absence of mmers on of resin piece formed of PP | | Presence | Presence | Absence |
| Residual rate (%) | | 84.5 | 74.9 | 831 |

As shown in the results listed in Table 1, from the comparison between Reference Examples 2 and 3, the content of netarsudil in the aqueous composition after stored under high temperature conditions was further reduced

due to the immersion of the pieces formed of polypropylene. Such a content reduction was caused by the immersion of the pieces formed of polypropylene, so that it is assumed that netarsudil was adsorbed to the pieces formed of polypropylene.

Meanwhile, from the comparison between Reference Examples 1 and 2, it was revealed that the content reduction of netarsudil was suppressed when the aqueous composition comprising netarsudil further comprised a boric acid

From the test results above, it was revealed that if the aqueous composition comprising the compound represented by the formula (1) exemplified by netarsudil or a salt thereof or a solvate thereof is further comprised of an acid such as borate exemplified by a boric acid, the content reduction (adsorption) which may occur in the aqueous composition housed in a container formed of a polyolefin resin exemplified by polypropylene after storage under high temperatures can be relatively suppressed.

[Test Example 2] Adsorption Inhibition Test 2

The procedures of Test Example 1 were repeated with the exception that the formulation of the aqueous composition was changed as shown in Table 2, thereby performing this test.

The results are shown in Table 2.

[Table 2]

| | | Reference Example 4 | Reference Example 5 | Reference Example 6 |
|---|---|---|---|---|
| Aqueous composton (amount per 100 mL) | Netarsudil dimesylate | 0.02848 g (002 g in terms of a free form) | 0.02848 g (002 g in terms of a free form) | 0.02848 g (002 g in terms of a free form) |
| | Disodium edetate hydrate | 0.01 g | - | - |
| | Sodium hydroxide | q.s. (pH 5.0) | q.s. (pH 5.0) | q.s. (pH 5.0) |
| | Purified water | Balance | Balance | Balance |
| Presence or absence of mmers on of resin piece formed of PP | | Presence | Presence | Absence |
| Residual rate (%) | | 83.9 | 74.9 | 83.1 |

As shown in the results listed in Table 2, from the comparison between Reference Examples 4 and 5, it was revealed that the content reduction of netarsudil was suppressed when the aqueous composition comprising netarsudil further comprised disodium edetate hydrate.

From the test results above, it was revealed that if the aqueous composition comprising the compound represented by the formula (1) exemplified by netarsudil or a salt thereof or a solvate thereof is further comprised of an acid such as aliphatic carboxylate exemplified by disodium edetate hydrate, the content reduction (adsorption) which may occur in the aqueous composition housed in a container formed of a polyolefin resin exemplified by polypropylene after storage under high temperatures can be relatively suppressed.

[Test Example 3] Adsorption Inhibition Test 3

The procedures of Test Example 1 were repeated with the exception that the formulation of the aqueous composition was changed as shown in Table 3, thereby performing this test.

The results are shown in Table 3.

[Table 3]

| | | Reference Example 7 | Reference Example 8 | Reference Example 9 |
|---|---|---|---|---|
| Aqueous composton (amount per 100 mL) | Netarsud dimesylate | 0.02848 g (002 g in terms of a free form) | 0.02848 g (002 g in terms of a free form) | 0.02848 g (002 g in terms of a free form) |
| | Benza kon um chloride | 0.015 g | - | - |
| | Sodium hydroxide | q.s. (pH 50) | q.s. (pH 50) | q.s. (pH 50) |
| | Purified water | Balance | Balance | Balance |
| Presence or absence of mmers on of resin piece formed of PP | | Presence | Presence | Absence |
| Residual rate (%) | | 85.4 | 74.9 | 831 |

As shown in the results listed in Table 3, from the comparison between Reference Examples 7 and 8, it was revealed that the content reduction of netarsudil was suppressed when the aqueous composition comprising netarsudil further comprised benzalkonium chloride.

From the test results above, it was revealed that if the aqueous composition comprising the compound represented by the formula (1) exemplified by netarsudil or a salt thereof or a solvate thereof is further comprised of a quaternary ammonium type surfactant exemplified by benzalkonium chloride, the content reduction (adsorption) which may occur in the aqueous composition housed in a container formed of a polyolefin resin exemplified by polypropylene after storage under high temperatures can be relatively suppressed.

[Test Example 4] Adsorption Inhibition Test 4

The procedures of Test Example 1 were repeated with the exception that the formulation of the aqueous composition was changed as shown in Table 4, thereby performing this test.

The results are shown in Table 4.

[Table 4]

| | | Reference Example 10 | Reference Example 11 | Reference Example 12 |
|---|---|---|---|---|
| Aqueous composition (amount per 100 mL) | Netarsud dimesylate | 0.02848 g (002 g in terms of a free form) | 0.02848 g (002 g in terms of a free form) | 0.02848 g (002 g in terms of a free form) |
| | Dimannito | 4.7 g | - | - |
| | Sodium hydroxide | q.s. (pH 50) | q.s. (pH 5.0) | q.s. (pH 50) |
| | Purified water | Balance | Balance | Balance |
| Presence or absence of mmers on of resin piece formed of PP | | Presence | Presence | Absence |
| Residual rate (%) | | 881 | 74.9 | 831 |

As shown in the results listed in Table 4, from the comparison between Reference Examples 10 and 11, it was revealed that the content reduction of netarsudil was suppressed when the aqueous composition comprising netarsudil further comprised D-mannitol.

From the test results above, it was revealed that if the aqueous composition comprising the compound represented by the formula (1) exemplified by netarsudil or a salt thereof or a solvate thereof is further comprised of a polyhydric alcohol exemplified by D-mannitol, the content reduction (adsorption) which may occur in the aqueous composition housed in a container formed of a polyolefin resin exemplified by polypropylene after storage under high temperatures can be relatively suppressed.

[Test Example 5] Adsorption Inhibition Test 5

The procedures of Test Example 1 were repeated with the exception that the formulation of the aqueous composition was changed as shown in Table 5, thereby performing this test.

The results are shown in Table 5.

[Table 5]

| | | Reference Example 13 | Reference Example 14 | Reference Example 15 |
|---|---|---|---|---|
| Aqueous composition (amount per 100 mL) | Netarsud dimesylate | 0.02848 g (002 g in terms of a free form) | 0.02848 g (002 g in terms of a free form) | 0.02848 g (002 g in terms of a free form) |
| | Macrogol 400 | 2.5 g | - | - |
| | Sodium hydroxide | q.s. (pH 5.0) | q.s. (pH 5.0) | q.s. (pH 50) |
| | Purified water | Balance | Balance | Balance |
| Presence or absence of immersion of resin piece formed of PP | | Presence | Presence | Absence |
| Residual rate (%) | | 88.9 | 74.9 | 831 |

As shown in the results listed in Table 5, from the comparison between Reference Examples 13 and 14, it was revealed that the content reduction of netarsudil was suppressed when the aqueous composition comprising netarsudil further comprised macrogol 400.

From the test results above, it was revealed that if the aqueous composition comprising the compound represented by the formula (1) exemplified by netarsudil or a salt thereof or a solvate thereof is further comprised of a polyhydric alcohol exemplified by macrogol 400, the content reduction (adsorption) which may occur in the aqueous composition housed in a container formed of a polyolefin resin exemplified by polypropylene after storage under high temperatures can be relatively suppressed.

[Test Example 6] Adsorption Inhibition Test 6

The procedures of Test Example 1 were repeated with the exception that the formulation of the aqueous composition was changed as shown in Table 6, thereby performing this test.

The results are shown in Table 6.

[Table 6]

| | | Reference Example 16 | Reference Example 17 | Reference Example 18 |
|---|---|---|---|---|
| Aqueous composition (amount per 100 mL) | Netarsudil dimesylate | 0.02848 g (002 g in terms of a free form) | 0.02848 g (002 g in terms of a free form) | 0.02848 g (002 g in terms of a free form) |
| | Latanoprost | 0005 g | - | - |
| | Sod um hydrox de | q.s. (pH 5.0) | q.s. (pH 5.0) | q.s. (pH 5.0) |
| | Purified water | Balance | Balance | Balance |
| Presence or absence of mmers on of resin piece formed of PP | | Presence | Presence | Absence |
| Residual rate (%) | | 87.4 | 74.9 | 831 |

As shown in the results listed in Table 6, from the comparison between Reference Examples 16 and 17, it was revealed that the content reduction of netarsudil was suppressed when the aqueous composition comprising netarsudil

further comprised latanoprost.

From the test results above, it was revealed that if the aqueous composition comprising the compound represented by the formula (1) exemplified by netarsudil or a salt thereof or a solvate thereof is further comprised of a prostaglandin compound exemplified by latanoprost, the content reduction (adsorption) which may occur in the aqueous composition housed in a container formed of a polyolefin resin exemplified by polypropylene after storage under high temperatures can be relatively suppressed.

[Test Example 7] Heat Stability Test

The heat stability of netarsudil in the aqueous composition was assessed by examining the presence or absence of content reduction of netarsudil after stored under high temperature for a certain period.

In other words, an aqueous composition having the formulation shown in Table 7 was prepared with an ordinary method, and then the obtained composition was housed in a container formed of low-density polyethylene (LDPE), high density-polyethylene (HDPE) or polypropylene (PP) to obtain pharmaceutical preparations. In addition, the same aqueous composition was housed in a container formed of glass to obtain a pharmaceutical preparation of Comparative Example.

Each of the obtained pharmaceutical preparations was stored at 80°C for a week. To examine the presence or absence of content reduction of netarsudil after storage, the concentrations of netarsudil in the aqueous composition before and after storage were measured according to the same method as in Test Example 1 for each of the pharmaceutical preparation.

Then, based on the calculated concentration of netarsudil in the aqueous composition, the residual rate (%) of netarsudil was assessed using the following expression:

Residual rate (%) = {(Concentration of netarsudil in the aqueous composition after storage)/(Concentration of netarsudil in the aqueous composition before storage)} × 100

The results are shown in Table 8.

[Table 7]

| Component | Amount (per 100 mL) |
|---|---|
| Netarsud dimesylate | 0.02848 g (002 g in terms of a free form) |
| Boric acid | 0.05 g |
| Sodium hydroxide | q.s. (pH 50) |
| Purified water | Balance |

[Table 8]

| | Material of container | After storage at 80°C for a week |
|---|---|---|
| Residual rate(%) | LDPE | 72.1 |
| | HDPE | 71.4 |
| | PP | 70.7 |
| | Comparative Example glass | 47.5 |

As shown in the results listed in Table 8, when the aqueous composition comprising netarsudil was housed in the container formed of polyethylene (LDPE, HDPE) or the container formed of polypropylene (PP), the content reduction due to storage under high temperature was suppressed relative to the case housed in a container formed of glass

(Comparative Example).

From the results of Test Example 7, it was revealed that, by housing the aqueous composition comprising the compound represented by the formula (1) exemplified by netarsudil or a salt thereof or a solvate thereof in a container formed of a polyolefin resin, the content reduction due to storage under high temperature was relatively suppressed and a pharmaceutical preparation having excellent storage stability was obtained

[Preparation Examples 1 to 36]

Pharmaceutical preparations of Preparation Examples 1 to 36 can be prepared by preparing aqueous compositions each comprising the components in amounts ((g) per 100 mL of the aqueous composition) shown in Tables 9 to 17 with an ordinary method, and then housing the prepared aqueous composition each in an eye drop container formed of high-density polyethylene.

[Table 9]

| | Preparat on Examples | | | |
|---|---|---|---|---|
| | 1 | 2 | 3 | 4 |
| Netarsud dimesylate | 0.02848 (0.02 in terms of a free form) | 0.02848 (0.02 in terms of a free form) | 0.02848 (0.02 in terms of a free form) | 0.02848 (0.02 in terms of a free form) |
| Boric acid | 0.05 | 0.3 | | |
| Borax | | 0.3 | | |
| Sodium dihydrogen phosphate | | | 0.031 | 0.5 |
| Disodium hydrogen phosphate | | | 0.07 | 0.8 |
| Citric acid | | | | |
| Sodium ctrate | | | | |
| Acetic acid | | | | |
| Sodium acetate | | | | |
| Disodium edetate | | 0.01 | 0.01 | |
| D-mannitol | 4.7 | | | |
| Glycerin | | 2.5 | | |
| Propylene glycol | | | 1.7 | |
| Xylitol | | | 0.7 | |
| Macrogol 6000 | | | | 1 |
| Macrogol 4000 | | | | 4 |
| Sorbitol | | | | |
| Trometamol | | | | |
| Benzalkonium chloride | 0.015 | 0.03 | 0.05 | |
| Benzododecinium bromide | | | | 0.01 |
| Benzethonium chloride | | | | 0.01 |
| Sodium chloride | | 0.1 | | |

(continued)

| | Preparat on Examples | | | |
|---|---|---|---|---|
| | 1 | 2 | 3 | 4 |
| Potassium chloride | | | | 0.2 |
| Calcium chloride | | | | |
| Sodium hydroxide | q.s. | q.s | q.s. | q.s. |
| Hydrochloric acid | q.s. | q.s | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 10]

| | Preparat on Examples | | | |
|---|---|---|---|---|
| | 5 | 6 | 7 | 8 |
| Netarsudil dimesylate | 0.02848 (002 in terms of a free form) | 0.02848 (002 in terms of a free form) | 0.02848 (002 in terms of a free form) | 0.02848 (002 in terms of a free form) |
| Boric acid | | | | |
| Borax | | | | |
| Sodium dihydrogen phosphate | | | | |
| Disodium hydrogen phosphate | | | | |
| Citric acid | 0.12 | 005 | | |
| Sodium citrate | 04 | 0.03 | | |
| Acetic acid | | | 0.001 | |
| Sodium acetate | | | 0.2 | 0.1 |
| Disodium edetate | | 0.01 | 0.01 | |
| D-mannitol | | | 1 | 3 |
| Glycerin | | | 3 | |
| Propylene glycol | | | | 1 |
| Xylitol | | | | |
| Macrogol 6000 | | | | |
| Macrogol 4000 | | | | |
| Sorbitol | 086 | | | |
| Trometamol | | 20 | | |
| Benzalkonium chloride | 0.015 | 0.03 | 0.05 | |
| Benzododecinium bromide | | | | 0.01 |

(continued)

| | Preparat on Examples | | | |
|---|---|---|---|---|
| | 5 | 6 | 7 | 8 |
| Benzethonium chloride | | | | 0.01 |
| Sodium chloride | | | 0.05 | |
| Potassium chloride | | | 0.05 | |
| Calcium chloride | 0.1 | | | |
| Sodium hydroxide | q.s. | q.s | q.s. | q.s. |
| Hydrochloric acid | q.s. | q.s | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 11]

| | Preparation Examples | | | |
|---|---|---|---|---|
| | 9 | 10 | 11 | 12 |
| Netarsudil dimesylate | 0.01424 (0.01 in terms of a free form | 0.05696 (004 in terms of a free form) | 0.02848 (0.02 in terms of a free form) | 0.02848 (0.02 in terms of a free form) |
| Boric acid | 0.05 | 0.3 | | |
| Borax | | 0.3 | | |
| Sodium dihydrogen phosphate | | | 0.031 | 0.5 |
| Disodium hydrogen phosphate | | | 0.07 | 0.8 |
| Citric acid | | | | |
| Sodium citrate | | | | |
| Acetic acid | | | | |
| Sodium acetate | | | | |
| Disodium edetate | | 0.01 | 0.01 | |
| D-mann to | 2 | 2 | | 2 |
| Glycerin | 2 | 2 | 1 | |
| Propylene glycol | | | | 1.5 |
| Xylitol | 0.3 | | | |
| Macrogol 6000 | 1 | | | |
| Macrogol 4000 | | 2 | | |
| Sorbito l | | | 0.5 | |
| Trometamo l | | | 10 | |
| Benzalkonium chloride | 0.015 | 0.03 | 0.05 | |

(continued)

| | Preparation Examples | | | |
|---|---|---|---|---|
| | 9 | 10 | 11 | 12 |
| Benzododecinium bromide | | | | 0.01 |
| Benzethonium chloride | | | | 0.01 |
| Sodium chloride | | 0.1 | | |
| Potassium chloride | | | | 02 |
| Calcium chloride | | | | |
| Sodium hydroxide | q.s. | q.s . | q.s. | q.s. |
| Hydrochloric acid | q.s. | q.s . | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 12]

| | Preparat on Examples | | | |
|---|---|---|---|---|
| | 13 | 14 | 15 | 16 |
| Netarsudil dimesylate | 0.02848 (0.02 in terms of a free form) | 0.02848 (0.02 in terms of a free form) | 0.01424 (0.01 in terms of a free form) | 0.05696 (0.04 in terms of a free form) |
| Boric acid | | | | |
| Borax | | | | |
| Sodium dihydrogen phosphate | | | | |
| Disodium hydrogen phosphate | | | | |
| Citric acid | 0.12 | 0.05 | | |
| Sodium citrate | 0.4 | 0.03 | | |
| Acetic acid | | | 0.001 | |
| Sodium acetate | | | 0.2 | 0.1 |
| Disodium edetate | | 0.01 | 0.01 | |
| D-mannitol | 3 | 1 | 1 | 2 |
| Glycerin | 1 | | 1 | |
| Propylene glycol | | | | 1 |
| Xylitol | | | | |
| Macrogol 6000 | | 1 | | |
| Macrogol 4000 | | 2 | | |
| Sorbitol | | | | 0.5 |
| Trometamol | | | 15 | |

(continued)

| | Preparat on Examples | | | |
|---|---|---|---|---|
| | 13 | 14 | 15 | 16 |
| Benzalkonium chloride | 0.015 | 0.03 | 0.05 | |
| Benzododecinium bromide | | | | 0.01 |
| Benzethonium chloride | | | | 0.01 |
| Sodium chloride | | | 0.05 | |
| Potassium chloride | | | 0.05 | |
| Calcium chloride | 01 | | | |
| Sodium hydroxide | q.s. | q s | q.s. | q.s. |
| Hydrochloric acid | q.s. | q s | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 13]

| | Preparat on Examples | | | |
|---|---|---|---|---|
| | 17 | 18 | 19 | 20 |
| Netarsud dimesylate | 0.02848 (0.02 in terms of a free form) | 0.02848 (0.02 in terms of a free form) | 0.02848 (0.02 in terms of a free form) | 0.02848 (0.02 in terms of a free form) |
| Latanoprost | 0.005 | | | |
| Travoprost | | 0.004 | | |
| Bimatoprost | | | 0.3 | |
| Tafuprost | | | | 0.0015 |
| Boric acid | 0.05 | 0.3 | | |
| Borax | | 0.3 | | |
| Sodium dihydrogen phosphate | | | 0.031 | 0.5 |
| Disodium hydrogen phosphate | | | 0.07 | 0.8 |
| Citric acid | | | | |
| Sodium ctrate | | | | |
| Acetic acid | | | | |
| Sodium acetate | | | | |
| Disodium edetate | 0.01 | 0.01 | 0.01 | 0.01 |
| D-mannitol | 4.7 | | | |
| Glycerin | | 2.5 | | |
| Propylene glycol | | | 1.7 | |
| Xylitol | | | 0.7 | |

(continued)

| | Preparat on Examples | | | |
|---|---|---|---|---|
| | 17 | 18 | 19 | 20 |
| Macrogo 400 | 2.5 | 1 | | 4 |
| Macrogol 4000 | | | | 1 |
| Sorbitol | | | | |
| Trometamol | | | | |
| Benzalkonium chloride | 0.2 | 0.03 | 0.05 | |
| Benzododecinium bromide | | | | 0.01 |
| Benzethonium chloride | | | | 0.01 |
| Polyoxyl 40 stearate (Myrj-52) | 05 | | | |
| Cremophor RH40 | | 0.5 | | |
| Polyoxyethylene hydrogenated castor of 40 | | | 0.5 | |
| Polysorbate 80 | | | | 0.5 |
| Sod um hydrox de | q.s. | q.s. | q.s. | q.s. |
| Hydrochlorid acid | q.s. | q.s. | q.s. | q.s. |
| Purified water | Total Amount 100 mL | Total Amount 100 mL | Total Amount 100 mL | Total Amount 100 mL |
| pH | 5.0 | 5 0 | 5 0 | 5.0 |

[Table 14]

| | Preparat on Examples | | | |
|---|---|---|---|---|
| | 21 | 22 | 23 | 24 |
| Netarsudil dimesylate | 0.02848 (002 in terms of a free form) | 0.02848 (002 in terms of a free form) | 0.02848 (002 in terms of a free form) | 0.02848 (002 in terms of a free form) |
| Latanoprost | 0.005 | | | |
| Travoprost | | 0.004 | | |
| Bimatoprost | | | 0.3 | |
| Tafuprost | | | | 0.0015 |
| Boric acid | | | | |
| Borax | | | | |
| Sodium dihydrogen phosphate | | | | |
| Disodium hydrogen phosphate | | | | |
| Citric acid | 0.12 | 0.05 | | |
| Sodium citrate | 0.4 | 0.03 | | |
| Acetic acid | | | 0.001 | |
| Sodium acetate | | | 0.2 | 0.1 |

(continued)

| | Preparat on Examples | | | |
|---|---|---|---|---|
| | 21 | 22 | 23 | 24 |
| Disodium edetate | | 0.01 | 0.01 | |
| D-mannitol | | | 1 | 3 |
| Glycerin | | | 3 | |
| Propylene glycol | | | | 1 |
| Xylitol | | | | |
| Macrogol 400 | | | | |
| Macrogol 4000 | | | | |
| Sorbitol | 0.86 | | | |
| Trometamol | | 20 | | |
| Benzalkonium chloride | 0.015 | 0.03 | 0.05 | |
| Benzododecinium bromide | | | | 0.01 |
| Benzethonium chloride | | | | 0.01 |
| Polyoxyl 40 stearate (Myrj-52) | 0.5 | | | |
| Cremophor RH40 | | 0.5 | | |
| Polyoxyethylene hydrogenated castor of 40 | | | 0.5 | |
| Polysorbate 80 | | | | 0.5 |
| Sod um hydroxide | q.s. | q.s | q.s. | q.s. |
| Hydrochlorid acid | q.s. | q.s | q.s. | q.s. |
| Purified water | Total Amount 100 mL | Total Amount 100 mL | Total Amount 100 mL | Total Amount 100 mL |
| pH | 6.0 | 5.5 | 6.0 | 5.5 |

[Table 15]

| | Preparat on Examples | | | |
|---|---|---|---|---|
| | 25 | 26 | 27 | 28 |
| Netarsudil dimesylate | 0.02848 (002 n terms of a free form) | 0.02848 (002 n terms of a free form) | 0.02848 (002 n terms of a free form) | (002 n terms of a free form) |
| Latanoprost | 0.005 | | | |
| Travoprost | | 0.004 | | |
| Bimatoprost | | | 0.3 | |
| Tafuprost | | | | 0.0015 |
| Boric acid | 0.05 | 0.3 | | |
| Borax | | 0.3 | | |

(continued)

| | Preparat on Examples | | | |
|---|---|---|---|---|
| | 25 | 26 | 27 | 28 |
| Sodium dihydrogen phosphate | | | 0.031 | 0.5 |
| Disodium hydrogen phosphate | | | 0.07 | 0.8 |
| Citric acid | | | | |
| Sodium citrate | | | | |
| Acetic acid | | | | |
| Sodium acetate | | | | |
| Disodium edetate | | 0.01 | 0.01 | |
| D≡mannitol | 2 | 2 | | 2 |
| Glycerin | 2 | 2 | 1 | |
| Propylene glycol | | | | 1.5 |
| Xylito l | 0.3 | | | |
| Macrogol 400 | | 2 | | |
| Macrogol 4000 | | 1 | | |
| Sorbito l | | | 0.5 | |
| Trometamo l | | | 10 | |
| Benzalkonium chloride | 0.015 | 0.03 | 0.05 | |
| Benzododecinium bromide | | | | 0.01 |
| Benzethonium chloride | | | | 0.01 |
| Polyoxyl 40 stearate (Myrj-52) | 05 | | | |
| Cremophor RH40 | | 0.5 | | |
| Polyoxyethylene hydrogenated castor of 40 | | | 0.5 | |
| Polysorbate 80 | | | | 0.5 |
| Sodium hydroxide | q.s. | q.s. | q.s. | q.s. |
| Hydrochloid acid | q.s. | q.s. | q.s. | q.s. |
| Purified water | Total Amount 100 mL | Total Amount 100 mL | Total Amount 100 mL | Total Amount 100 mL |
| pH | 7.0 | 6 0 | 7 0 | 6.0 |

[Table 16]

| | Preparation Examples | | | |
|---|---|---|---|---|
| | 29 | 30 | 31 | 32 |
| Netarsudi dimesylate | 0.02848 (002 in terms of a free form) | (002 in terms of a free form) | (002 in terms of a free form) | (002 in terms of a free form) |
| Latanoprost | 0.005 | | | |

(continued)

| | Preparation Examples | | | |
|---|---|---|---|---|
| | 29 | 30 | 31 | 32 |
| Travoprost | | 0004 | | |
| Bimatoprost | | | 03 | |
| Tafuprost | | | | 0.0015 |
| Boric acid | | | | |
| Borax | | | | |
| Sodium dihydrogen phosphate | | | | |
| Disodium hydrogen phosphate | | | | |
| Citric acid | 0.12 | 005 | | |
| Sodium ctrate | 04 | 0.03 | | |
| Acetic acid | | | 0.001 | |
| Sodium acetate | | | 0.2 | 01 |
| Disodium edetate | | 0.01 | 0.01 | |
| D-mannitol | 3 | 1 | 1 | 2 |
| Glycerin | 1 | | 1 | |
| Propylene glycol | | | | 1 |
| Xylitol | | | | |
| Macrogol 400 | | 2 | | |
| Macrogol 4000 | | 1 | | |
| Sorbitol | | | | 0.5 |
| Trometamo | | | 15 | |
| Benzalkonium chloride | 0.015 | 003 | 0.05 | |
| Benzododecinium bromide | | | | 0.01 |
| Benzethonium chloride | | | | 0.01 |
| Polyoxyl 40 stearate (Myrj-52) | 0.5 | | | |
| Cremophor RH40 | | 0.5 | | |
| Polyoxyethylene hydrogenated castor oil 40 | | | 0.5 | |
| Polysorbate 80 | | | | 05 |
| Sodium hydroxide | q.s. | q.s. | q.s. | q.s. |
| Hydrochloric acid | q.s. | q.s. | q.s. | q.s. |
| Purified water | Tota Amount 100 mL | Tota Amount 100 mL | Tota Amount 100 mL | Tota Amount 100 mL |

(continued)

| | Preparation Examples | | | |
|---|---|---|---|---|
| | 29 | 30 | 31 | 32 |
| pH | 6.0 | 5.5 | 6.0 | 5.5 |

[Table 17]

| | Preparat on Examp es | | | |
|---|---|---|---|---|
| | 33 | 34 | 35 | 36 |
| Netarsud dimesylate | (002 in terms of a free form) | (002 in terms of a free form) | (002 in terms of a free form) | (002 in terms of a free form) |
| Timolol maleate | 0 683 (0 5 mg in terms of a free form) | 0 683 (0 5 mg in terms of a free form) | 0.683 (0.5 mg in terms of a free form) | 0 683 (0 5 mg in terms of a free form) |
| Boric acid | 004 | 005 | | |
| Borax | 001 | | | |
| Sodium dihydrogen phosphate | | 0.031 | | |
| Disodium hydrogen phosphate | | 007 | | |
| Citric acid | | | 0.05 | |
| Sodium citrate | | | 0.03 | |
| Acetic acid | | | | 0.001 |
| Sodium acetate | | | | 02 |
| Disodium edetate | 001 | 001 | 001 | 001 |
| D-mannitol | 4.7 | | | |
| Glycerin | | 2.5 | | |
| Propylene glycol | | | 1.7 | |
| Xylitol | | | 07 | |
| Macrogol 400 | | 1 | | |
| Macrogol 4000 | | | 1 | |
| Sorbitol | | | | 3 |
| Trometamol | | | | 15 |
| Benzalkonium chloride | 0.015 | 003 | 0.05 | |
| Benzododecinium bromide | | | | 001 |
| Benzethonium chloride | | | | 001 |
| Methyl cellulose | 0.5 | 2 | | |
| Gellan gum | | | 02 | 06 |
| Sodium hydroxide | q.s. | q.s | q.s. | q.s. |
| Hydrochloric acid | q.s. | q.s | q.s. | q.s. |

(continued)

| | Preparat on Examp es | | | |
|---|---|---|---|---|
| | 33 | 34 | 35 | 36 |
| Purified water | Tota Amount 100 mL | Tota Amount 100 mL | Tota Amount 100 mL | Tota Amount 100 mL |
| pH | 5.0 | 6.0 | 7 0 | 6.0 |

[Preparation Examples 37 to 72]

Pharmaceutical preparations of Preparation Examples 37 to 72 can be prepared in the same way as in Preparation Examples 1 to 36 except for using an eye drop container formed of polypropylene instead of that formed of high-density polyethylene.

[Preparation Examples 73 to 108]

Pharmaceutical preparations of Preparation Examples 73 to 108 can be prepared in the same way as in Preparation Examples 1 to 36 except for using an eye drop container formed of cyclic polyolefin instead of that formed of high-density polyethylene.

[Preparation Examples 109 to 144]

Pharmaceutical preparations of Preparation Examples 109 to 144 can be prepared in the same way as in Preparation Examples 1 to 36 except for using an eye drop container formed of low-density polyethylene instead of that formed of high-density polyethylene.

[Preparation Examples 145 to 288]

Pharmaceutical preparations of Preparation Examples 145 to 288 can be prepared with an ordinary method by using 0.5 g of verosudil monohydrochloride in a free form instead of netarsudil dimesylate in the Preparation Examples 1 to 144.

[Preparation Examples 289 to 432]

Pharmaceutical preparations of Preparation Examples 289 to 432 can be prepared with an ordinary method by using 0.7 g of verosudil monohydrochloride in a free form instead of netarsudil dimesylate in the Preparation Examples 1 to 144.

[Preparation Examples 433 to 576]

Pharmaceutical preparations of Preparation Examples 433 to 576 can be prepared with an ordinary method by using 0.02 g of a compound represented by the formula (8) instead of netarsudil dimesylate in the Preparation Examples 1 to 144.

Industrial applicability

According to the present invention, a pharmaceutical preparation having excellent stability can be provided, and it can be suitably used in the pharmaceutical industry and the like.

## Claims

**1.** Use of latanoprost and a container formed of polypropylene or polyethylene for suppressing the content reduction of netarsudil or a salt thereof or a solvate thereof in a pharmaceutical preparation comprising an aqueous composition comprising said latanoprost and said netarsudil or a salt thereof or a solvate thereof, wherein the aqueous composition is housed in said container formed of polypropylene or polyethylene.

2. The use according to claim 1, wherein the polyolefin resin is polyethylene.

3. The use according to claim 1, wherein netarsudil or a salt thereof or a solvate thereof is netarsudil dimesylate.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012525386 A
- WO 2009091898 A
- JP 2016515520 A


### Non-patent literature cited in the description


- **BACHARACH J. et al.** *Ophthalmology,* 2015, vol. 122 (2), 302-7
- **STURDIVANT JM et al.** *Bioorg Med Chem Lett.,* 2016, vol. 26 (10), 2475-80
- **WILLIAMS RD et al.** *Am- J-Ophthalmol,* 2011, vol. 152 (5), 834-41